(19) Europäisches Patentamt
European Patent Office
Office européen des brevets

(11) EP 0 977 878 B1

(12) EUROPÄISCHE PATENTSCHRIFT

(45) Veröffentlichungstag und Bekanntmachung des Hinweises auf die Patenterteilung:
**20.07.2005 Patentblatt 2005/29**

(21) Anmeldenummer: **98924224.3**

(22) Anmeldetag: **27.04.1998**

(51) Int Cl.[7]: **C12N 15/82**, C12N 9/00, C12N 1/19, C07K 16/16, C12Q 1/68, G01N 33/53, A01H 5/00

(86) Internationale Anmeldenummer:
**PCT/EP1998/002483**

(87) Internationale Veröffentlichungsnummer:
**WO 1998/049330 (05.11.1998 Gazette 1998/44)**

(54) **DNA-SEQUENZEN KODIEREND FÜR DIE UNTEREINHEIT CHLD PFLANZLICHER MAGNESIUM-CHELATASEN SOWIE VERFAHREN ZUR AKTIVITÄTSBESTIMMUNG PFLANZLICHER MAGNESIUM-CHELATASEN**

DNA SEQUENCES CODING FOR SUBUNIT CHLD OF PLANT MAGNESIUM CHELATASES FOR DETERMINING THEIR ACTION

SEQUENCES D'ADN CODANT POUR LA SOUS-UNITE CHLD DE CHELATASES VEGETALES DE MAGNESIUM POUR DETERMINER LEURS EFFETS

(84) Benannte Vertragsstaaten:
**DE FR GB NL**

(30) Priorität: **25.04.1997 DE 19717656**

(43) Veröffentlichungstag der Anmeldung:
**09.02.2000 Patentblatt 2000/06**

(73) Patentinhaber: **Bayer CropScience GmbH
65929 Frankfurt/Main (DE)**

(72) Erfinder:
- **GRIMM, Bernhard
D-06466 Gatersleben (DE)**
- **PAPENBROCK, Jutta
D-30419 Hannover (DE)**
- **HÄNEL, Frank
D-07745 Jena (DE)**
- **GRÄFE, Susanna
D-07745 Jena (DE)**
- **SCHMIDT, Frank
D-60598 Frankfurt (DE)**
- **STREBER, Wolfgang
D-65812 Bad Soden (DE)**

(56) Entgegenhaltungen:
- **JENSEN P.E. ET AL: 'Structural genes of Mg-chelatase subunits in barley: Xantha-f, -g, -h' MOLECULAR AND GENERAL GENETICS Bd. 250, 1996, Seiten 383 - 394, XP002076472**
- **GIBSON L.C.D. ET AL: 'A putative Mg chelatase subunit from Arabidopsis thaliana cv C24' PLANT PHYSIOLOGY Bd. 111, 1996, Seiten 61 - 71, XP002076474**
- **NAKAYAMA M. ET AL: 'Cloning, subcellular localisation, and expression of CHLI, a subunit of magnesium-chelatase in soybean' BIOCHEMICAL AND BIOPHYSICAL RESEARCH COMMUNICATIONS Bd. 215, Nr. 1, 1995, Seiten 422 - 428, XP002076475**
- **KANNANGARA C.G. ET AL: 'Magnesium chelatase: association with ribosomes and mutant complementation studies identify barley subunit Xantha-G as a functional counterpart of Rhodobacter subunit BchD' MOLECULAR AND GENERAL GENETICS Bd. 254, M{rz 1997, Seiten 85 - 92, XP002076473**
- **GIBSON L.C.D. ET AL: 'Magnesium-protoporphyrin chelatase of Rhodobacter sphaeroides: reconstitution of activity by combining the products of the bchH, -I, and -D genes expressedin Escherichia coli' PROCEEDINGS OF THE NATIONAL ACADEMY OF SCIENCES OF THE USA Bd. 92, M{rz 1995, Seiten 1941 - 1944, XP002076476**
- **HUDSON A. ET AL: 'Olive: a key gene required for chlorophyll biosynthesis in Antirrhinum majus' THE EMBO JOURNAL Bd. 12, Nr. 10, 1993, Seiten 3711 - 3719, XP002076477**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 977 878 B1

- KONCZ C. ET AL: 'Isolation of a gene encoding a novel chloroplast protein by T-DNA tagging in Arabidopsis thaliana' THE EMBO JOURNAL Bd. 9, Nr. 5, 1990, Seiten 1337 - 1346, XP002076478
- PAPENBROCK J. ET AL: 'Mg-chelatase of tobacco: identification of a Chl D cDNA sequence encoding a third subunit, analysis of the interaction of the three subunits with the yeast two-hybrid system, and reconstitution of the enzyme activity by co-expression of recombinant CHL D, CHL H and CHL I' THE PLANT JOURNAL Bd. 12, Nr. 5, November 1997, Seiten 981 - 990, XP002076479
- PAPENBROCK J. ET AL: 'Untitled' EMBL SEQUENCE DATA LIBRARY 01 Januar 1998, HEIDELBERG, GERMANY, XP002076480
- PAPENBROCK J. ET AL: 'Identification of a plant ChlD cDNA sequence homologous to a bacterial gene encoding a third subunitof Mg-chelatase' EMBL SEQUENCE DATA LIBRARY 01 Juli 1997, XP002076481
- LUO M. ET AL: 'Cloning and sequencing of a cDNA encoding the putative Mg-chelatase subunit D (Accession No. AF014399) from Pea (Pisum sativum L. cv Spring)' PLANT PHYSIOLOGY Bd. 115, Nr. 1, September 1997, Seite 315, XP002076482
- KRUSE E. ET AL: 'Isolation and characterisation of tobacco (Nicotiana tabacum) cDNA clones encoding proteins involved in Magnesium chelation into protoporphyrin IX' PLANT MOLECULAR BIOLOGY Bd. 35, Dezember 1997, Seiten 1053 - 1056, XP002076483
- JENSEN P.E. ET AL: 'Expression of the chlI, chlD, and chlH genes from the Cyanobacterium synechocystis PCC6803 in Escherichia coli and demonstration that the three cognate proteins are required for magnesium-protoporphyrin chelatase activity.' THE JOURNAL OF BIOLOGICAL CHEMISTRY Bd. 271, Nr. 28, 12 Juli 1996, Seiten 16662 - 16667

## Beschreibung

Die vorliegende Erfindung betrifft die Untereinheit CHLD pflanzlicher Magnesium-Chelatase (Mg-Chelatase), DNA Sequenzen kodierend für die Untereinheit CHLD pflanzlicher Mg-Chelatase, Verfahren zur Herstellung der Untereinheit CHLD der pflanzlichen Mg-Chelatase, Verfahren zur Aktivitätsbestimmung pflanzlicher Mg-Chelatase sowie transgene Pflanzen, die mit der erfindungsgemäßen Mg-Chelatase-DNA transformiert wurden.

Die Chlorophylle haben als Cofaktoren des Photosystems Anteil an der Umwandlung von Licht in chemische Energie und sind somit notwendig für das Wachstum und das Überleben von Pflanzen.

Während der Biosynthese der Chlorophylle erfolgt der Einbau des Magnesiums in das Porphyrin mit Hilfe eines membranassoziierten Enzyms, der Mg-Chelatase, die aus mehreren Untereinheiten aufgebaut ist.

Es ist bereits bekannt, daß die bakterielle Mg-Chelatase aus drei Untereinheiten (D, H und I) besteht, deren korrespondierenden Gensequenzen mit bchD, bchH und bchI bezeichnet wurden (Burke et al. (1993), J. Bacteriol. 175, 2414-2422; Coomber et al. (1990), Mol. Microbiol. 4, 977-989; Gibson et al. (1995), Proc. Natl. Acad. Sci. USA, 92, 1941-1844; Jensen et al. (1996), J. Biol. Chem. 271, 16662-16667).

Tabelle I:

| Auflistung der Gene bekannter Mg-Chelatase Untereinheiten | | | | |
|---|---|---|---|---|
| Rhodobacter capsulatus | Rhodobacter sphaeroides | Synechocystis PCC 6803 | Arabidopsis thaliana | Antirrhinium majus |
| Burke et al. | Coomber et al. | Jensen et al. | Koncz et al. | Hudson et al. |
| bchD | bchD | chlD | | |
| bchH | bchH | chlH | chlH | olive |
| bchI | bchI | chlI | ch42 | 3DchI |

In bezug auf pflanzliche Mg-Chelatasen wurden bisher zwei Untereinheiten beschrieben, die offenbar den bakteriellen Mg-Chelatase-Untereinheiten bchH und bchI entsprechen (Koncz et al., (1990), EMBO J. 9, 1337-1346; Hudson et al., (1993), EMBO J. 12, 3711-3719; Eibson et al., (1996), Plant Physiol. 121, 61-71). Bislang ist nicht bekannt, welche weiteren Untereinheiten am Aufbau der pflanzlichen Mg-Chelatase beteiligt sind. Mit den beiden bekannten pflanzlichen Untereinheiten CHLI und CHLH konnte weder alleine, noch zusammen mit den bekannten bakteriellen Untereinheiten vom Typ D (CHLD bzw BCHD) eine Enzymaktivität festgestellt werden.

Aufgrund ihrer Schlüsselstellung innerhalb der Chlorophyllbiosynthese stellt die pflanzliche Mg-Chelatase einen grundlegend neuen Angriffspunkt für die Entwicklung einer neuen Generation hochspezifisch wirksamer, herbizider Verbindungen dar. Darüber hinaus kann durch eine Beeinflussung der Genexpression (Suppression, Überexpression), der natürlichen oder (z.B. gentechnisch) modifizierten Expressionsprodukte der Mg-Chelatase oder auch durch spezifische Mg-Chelatase-Inhibitoren in hohem Maße Einfluß auf die Vitalität und/oder das Wachstum von phototrophen ein- und mehrzelligen Organismen, insbesondere Bakterien, Algen und Pflanzen genommen werden.

Die enzymatische Aktivität der pflanzlichen Mg-Chelatase wurde ursprünglich an intakten Chloroplasten gemessen (Castelfranco et al., (1979) Arch. Biochem.

Biophys. 192, 592-598; Fuesler et al. (1982) Plant Physiol. 69, 421-423). Inzwischen wurden Aktivitätsbestimmungen auch an aufgebrochenen Plastiden (Walker et al. (1991) Proc. Natl. Acad. Sci. 88, 5789-5793) sowie subplastidären Membranfraktionen vorgenommen (Lee et al., (1992), Plant Physiol. 99, 1134-1140).

Es wurde nun überraschenderweise eine für eine Untereinheit des Enzyms der pflanzlichen Mg-Chelatase kodierende DNA gefunden. Im folgenden wird die DNA-Sequenz als chlD und die Aminosäuresequenz als CHLD bezeichnet.

Des weiteren wurde gefunden, daß eine Untereinheit CHLD der pflanzlichen Mg-Chelatase überraschenderweise geeignet ist, zusammen mit den Untereinheiten CHLI und CHLH eine funktionell intakte, d.h. enzymatisch aktive, pflanzliche Mg-Chelatase zu rekonstituieren; so daß die erfindungsgemäße pflanzliche Mg-Chelatase-Untereinheit CHLD neuartige Testverfahren (in-vivo und in-vitro) der pflanzlichen Mg-Chelatase-Aktivität bereitstellt.

Gegenstand der vorliegenden Erfindung ist daher ein Nukleinsäuremolekül, dadurch gekennzeichnet, daß es für ein Protein mit der Funktion einer pflanzlichen Mg-Chelatase-Untereinheit CHLD kodiert, vorzugsweise ein Nukleinsäuremolekül gemäß SEQ ID Nr. 1, ein biologisch aktives Fragment daraus sowie komplementäre oder anti-sense-Sequenzen.

Die Erfindung betrifft pflanzliche chlD-Sequenzen, vorzugsweise aus dicotylen Pflanzen, besonders bevorzugt Solanaceae und insbesondere Nicotiana tabacum.

Ein weiterer Gegenstand der vorliegenden Erfindung ist ein Nukleinsäuremolekül, dadurch gekennzeichnet, daß es für ein Protein gemäß SEQ ID Nr. 2 mit der Funktion einer pflanzlichen Mg-Chelatase-Untereinheit CHLD oder ein biologisch aktives Fragment daraus kodiert oder es eine komplementäre oder anti-sense-Sequenz zu einem solchen Nukleinsäuremolekül bildet.

"Spezifisch Hybridisieren" bezeichnet im allgemeinen die Eigenschaft eines einzelsträngigen Nukleinsäuremoleküls mit einem komplementären Nukleinsäuremolekül Wasserstoffbrücken, Basenpaare und gegebenenfalls einen Doppelstrang auszubilden.

Unter dem Begriff "rekombinanter Organismus" ist die Zelle eines durch in vitro veränderter DNA oder durch Integration von DNA modifizierten Organismus zu verstehen, beispielsweise von rekombinante Hefe-, Bakterien-, Algen, Insekten- oder Pflanzenzellen.

Die kleine Schreibweise "chl" bezeichnet konventionsgemäß die Gene der Mg-Chelatase-Untereinheiten D, I, und H, wohingegen die Großschrift "CHL" die Genprodukte, d.h. Proteine, bezeichnet.

Erfindungsgegenstand ist auch die Verwendung der erfindungsgemäßen Nukleinsäuremoleküle zur Herstellung von Antikörpern gegen deren Expressionsprodukte.

Gegenstand der Erfindung sind auch nicht-natürlich vorkommende chimäre Gene, enthaltend einen Promotor, der funktionell mit einem erfindungsgemäßen DNA-Molekül fusioniert ist.

Gegenstand der Erfindung ist auch ein Vektor, vorzugsweise ein rekombinanter Vektor, enthaltend ein erfindungsgemäßes Nukleinsäuremolekül enthaltend ein chimäres Gen enthaltend einen Promotor, der funktionell mit einem erfindungsgemäßen DNA-Molekül fusioniert ist.

Gegenstand der Erfindung ist auch eine rekombinante Wirtszelle, dadurch gekennzeichnet, daß diese Wirtszelle ein erfindungsgemäßes DNA-Molekül exprimiert, vorzugsweise daß diese Wirtszelle stabil mit einem rekombinanten Vektor, enthaltend ein chimäres Gen enthaltend einen Promotor, der funktionell mit einem erfindungsgemäßen Nukleinsäuremolekül fusioniert ist, transformiert ist oder nach anderen, dem Fachmann bekannten Transformationsverfahren transformiert ist und ein erfindungsgemäßes DNA Molekül exprimiert.

Erfindungsgegenstand ist ebenfalls die Verwendung der erfindungsgemäßen Nukleinsäuremoleküle zur Herstellung von transgenen Pflanzen.

Gegenstand der Erfindung sind auch transgene Pflanzen, transgene Pflanzenzellen, transgene Pflanzenteile, ransgene Pflanzensamen, transgenes Vermehrungsgut, enthaltend ein erfindungsgemäßes Nukleinsäuremolekül.

Gegenstand der Erfindung sind auch Verfahren zur Herstellung transgener Pflanzen, transgener Pflanzenzellen, transgener Pflanzenteile, transgener Pflanzensamen, transgenen Vermehrungsguts mittels einer rekombinanten Wirtszelle, dadurch gekennzeichnet, daß diese Wirtszelle mit einem erfindungsgemäßen DNA-Molekül transformiert ist.

Die Erfindung betrifft ebenfalls Vermehrungsmaterial der erfindungsgemäßen Pflanzen, beispielsweise Früchte, Samen, Knollen, Wurzelstöcke, Sämlinge und Stecklinge.

Als Empfänger-Pflanzen für ein erfindungsgemäßes Gen kommen alle landwirtschaftlich wichtigen monokotylen und dikotylen Kulturpflanzen in Betracht, vorzugsweise Mais und andere Getreide, wie beispielsweise Weizen, Roggen, Gerste, Hirse, Hafer, Maniok und Reis sowie Baumwolle, Tabak, Zuckerrüben, Zuckerrohr, Kartoffeln, Raps, Sonnenblumen, Soja oder Obst- und Gemüsepflanzen.

Zur Expression der erfindungsgemäßen Nukleinsäuremoleküle in pflanzlichen Zellen werden diese mit regulatorischen DNA-Elementen verknüpft, die die Transkription in pflanzlichen Zellen gewährleisten. Hierzu zählen insbesondere Promotoren. Generell kommt für die Expression jeder in pflanzlichen Zellen aktive Promotor in Frage.

Der Promotor kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein. Geeignete Promotoren sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., EMBO J. 8 (1989), 23-29) für eine sink-spezifische Expression (z. B. Kartoffelknollen, Rüben, Tomatenfrucht) oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451), alle in Plastiden konstitutiv aktiven Promotoren z.B. die Expressions-Signalsequenzen der *psbA*-Kassette (Staub & Maliga (1993) EMBO Journal 12: 601-606; Zoubenko et al. (1994) Nucleic Acids Research 22:3819-3824) und der *Prn* Promotor (Svab & Maliga (1993) Proc. Natl. Acad. Sci. USA 90: 913-917) oder für eine endospermspezifische Expression der HMG-Promotor aus Weizen, der USP-Promotor, der Phaseolinpromotor oder Promotoren von Zein-Genen aus Mais. Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. Gielen et al., EMBO J. 8 (1989), 23-29) und sind im allgemeinen beliebig austauschbar.

Zur Vorbereitung der Einführung fremder Gene in höhere Pflanzen stehen eine große Anzahl von Klonierungsvektoren zur Verfügung, die ein Replikationssignal für E. coli und ein Markergen zur Selektion transformierter Bakterienzellen enthalten. Beispiele für derartige Vektoren sind pBR322, pUC-Serien, M13mp-Serien, pACYC184. Die gewünschte Sequenz kann an einer passenden Restriktionsschnittstelle in den Vektor eingeführt werden. Das erhaltene Plasmid wird für die Transformation von E. coli-Zellen verwendet. Transformierte E. coli-Zellen werden in einem geeigneten Medium gezüchtet, anschließend geerntet und lysiert. Das Plasmid wird wiedergewonnen. Als Analysemethode zur Charakterisierung der gewonnenen Plasmid-DNA werden im allgemeinen Restriktionsanalysen, Gelelektrophoresen und weitere biochemischmolekularbiologische Methoden eingesetzt. Nach jeder Manipulation kann die Plasmid-DNA gespalten und gewonnene DNA-Fragmente mit anderen DNA-Sequenzen verknüpft werden. Jede Plasmid-DNA-Sequenz kann in den gleichen oder anderen Plasmiden kloniert werden.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, die Injektion, die Elektroporation von DNA, das Einbringen von DNA mittels der biolistischen Methode etc..

Bei der Injektion und Elektroporation von DNA in Pflanzenzellen werden an sich keine speziellen Anforderungen an die verwendeten Plasmide gestellt. Es können einfache Plasmide wie z.B. PUC-Derivate verwendet werden. Sollen aber aus derartig transformierten Zellen ganze Pflanzen regeneriert werden, ist die Anwesenheit eines selektierbaren Markergens notwendig.

Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.

Werden für die Transformation Agrobakterien verwendet, muß die einzuführende DNA in spezielle Plasmide kloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E. coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al. Mol. Gen. Genet. 163 (1978), 181-187). Das als Wirtszelle dienende Agrobakterium soll ein Plasmid enthalten, das eine vir-Region trägt. Die vir-Region ist für den Transfer der T-DNA in die Pflanzenzelle notwendig; zusätzliche T-DNA kann vorhanden sein. Das derartig transformierte Agrobakterium wird zur Transformation von Pflanzenzellen verwendet.

Die Verwendung von T-DNA für die Transformation von Pflanzenzellen ist ausführlich in EP 120 516 beschrieben; Hoekema, in: The Binary Plant Vector System, Offsetdrukkerij Kanters B.V., Alblasserdam (1985), Chapter V; Fraley et al., Crit. Rev. Plant. Sci., 4, 1-46 und An et al. EMBO J. 4 (1985), 277-287.

Für den Transfer der DNA in die Pflanzenzelle können Pflanzen-Explantate zweckmäßigerweise mit Agrobacterium tumefaciens oder Agrobacterium rhizogenes kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z.B. Willmitzer, L., 1993 Transgenic Plants. In: Biotechnology, A Multi-Volume Comprehensive Treatise (H.J. Rehm, G. Reed, A. Pühler, P. Stadler, eds.), Vol. 2, 627-659, VCH Weinheim).

Alternative Systeme zur Transformation von monokotylen Pflanzen sind die Transformation mittels des biolistischen Ansatzes, die elektrisch oder chemisch induzierte DNA-Aufnahme in Protoplasten, die Elektroporation von partiell permeabilisierten Zellen, die Makroinjektion von DNA in Blütenstände, die Mikroinjektion von DNA in Mikrosporen und Pro-Embryonen, die DNA-Aufnahme durch keimende Pollen und die DNA-Aufnahme in Embryonen durch Quellung (zur Übersicht: Potrykus, Physiol. Plant (1990), 269 - 273).

Während die Transformation dikotyler Pflanzen über Ti-Piasmid-Vektorsysteme mit Hilfe von Agrobacterium tumefaciens wohl etabliert ist, weisen neuere Arbeiten darauf hin, daß auch monokotyle Pflanzen der Transformation mittels Agrobacterium basierender Vektoren sehr wohl zugänglich sind (Chan et al., Plant Mol. Biol. 22 (1993), 491-506; Hiei et al., Plant J. 6 (1994), 271-282; Bytebier et al., Proc. Natl. Acad. Sci. USA 84 (1987), 5345 - 5349; Raineri et al., Bio/Technology 8 (1990), 33 - 38; Gould et al., Plant Physiol. 95 (1991), 426 - 434; Mooney et al., Plant Cell Tiss. & Org. Cult. 25 (1991), 209 - 218; Li et al., Plant Mol. Biol. 20 (1992), 1037 - 1048).

Drei der oben genannten Transformationssysteme konnten in der Vergangenheit für verschiedene Getreide etabliert werden: die Elektroporation von Gewebe, die Transformation von Protoplasten und der DNA-Transfer durch Partikel-Beschuß in regenerierbare Gewebe und Zellen (Jähne et al., Euphytica 85 (1995), 35 - 44).

Die Transformation von Weizen wird in der Literatur verschiedentlich beschrieben (zur Übersicht: Maheshwari et al., Critical Reviews in Plant Science 14 (2) (1995), 149 - 178), vgl. auch Hess et al. (Plant Sci. 72 (1990), 233), Vasil et al. (Bio/Technology 10 (1992), 667 - 674), Weeks et al. (Plant Physiol. 102 (1993), 1077 - 1084) sowie Becker et al. (Plant J. 5(2) (1994), 299 - 307).

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid, wie Phosphinothricin, oder einem Antibiotikum, wie Kanamycin, G 418, Bleomycin oder Hygromycin, vermittelt. Der individuelle gewählte Marker sollte daher die Selektion transformierter Zellen gegenüber Zellen, denen die eingeführte DNA fehlt, gestatten.

Die transformierten Zellen wachsen innerhalb der Pflanze in der üblichen Weise (siehe auch McCormick et al., Plant Cell Reports 5 (1986), 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften. Von den Pflanzenzellen können Samen gewonnen werden.

Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind.

Gegenstand der Erfindung sind darüber hinaus Verfahren zur Herstellung eines erfindungsgemäßen Nukleinsäuremoleküls, dadurch gekennzeichnet, daß ein erfindungsgemäßes Nukleinsäuremolekül nach einem an sich bekannten Verfahren zur Herstellung von Nukleinsäuren hergestellt wird. Dem in der Klonierung von cDNA erfahrenen Fachmann stehen hierzu eine Vielzahl verschiedener Methoden zur Isolierung und Klonierung von Gensequenzen zur Verfügung.

Darüber hinaus können die erfindungsgemäßen Sequenzinformationen auch für eine Vielzahl anderer, dem Fachmann bekannter Methoden genutzt werden, wie z.B. der Durchmusterung von Expressionsbibliotheken zur Genisolierung mit Antikörpern oder auch zur Herstellung von Antikörpern (polyklonal oder monoklonal).

Für solche Methoden können die gesamte pflanzliche chlD-cDNA, vorzugsweise chlD aus Nicotiana tabacum gemäß SEQ ID Nr.1, Teilsequenzen aus SEQ ID Nr.1 oder aus SEQ ID Nr.1 abgeleitete Oligonukleotide als Sonden eingesetzt werden, gegebenenfalls solche, welche selektiv mit anderen CHLD-kodierenden Sequenzen aus einer Bank von klonierten Genfragmenten eines ausgewählten Organismus z.B. phototrophe Mikroorganismen oder mono- oder dikotyle Pflanzen hybridisieren.

Solche Techniken schließen beispielsweise auch die Durchmusterung von genomischen oder cDNA-Bibliotheken, kloniert in geeigneten Zellen oder Viren sowie die Amplifizierung durch Polymerase-Kettenreaktion (PCR) unter Verwendung von aus SEQ ID NR. 1 abgeleiteten Oligonukleotid-Primern ein.

Die isolierten chlD-(Teil)-Sequenzen gemäß vorliegender Erfindung, können außerdem durch gentechnische Standard-Methoden verändert oder erweitert werden, um gewünschte Eigenschaften zu erhalten. Um eine selektive Hybridisierung unter in vitro oder in vivo Bedingungen zu erhalten, sind chlD-spezifische Sonden mindestens 10 Nukleotide lang, vorzugsweise mindestens 17 Nukleotide lang.

Hybridisierungssonden gemäß vorliegender Erfindung können beispielsweise genutzt werden, um CHLD-kodierende Sequenzen (d.h. CHLD-kodierende Nukleinsäuremoleküle) aus einem ausgewählten Organismus mittels der dem Fachmann bekannten Methode der PCR zu amplifizieren oder zu analysieren und können des weiteren zur Isolierung von CHLD-kodierenden Sequenzen aus anderen Organismen oder als diagnostisches Mittel zum Nachweis von CHLD-kodierenden Sequenzen in anderen Organismen genutzt werden oder, um modifizierte CHLD-kodierende Sequenzen eines besonderen Phänotyps wie z. B. Herbizidresistenz, verändertem Chlorophyllgehalt, veränderter Fitness, verändertem Ertrag usw. zu identifizieren und zu isolieren.

Die chlD-spezifischen Hybridisierungssonden können auch verwendet werden, um unter Einsatz von Standard-Methoden den Genort eines natürlich vorkommenden chlD-Gens im Genom einer Pflanze zu kartieren. Diese Methoden umfassen, u.a. die Identifizierung von DNA-Polymorphismen und den Einsatz solcher Polymorphismen zur Analyse der Segregation von chlD-Genen relativ zu anderen Markern mit bekanntem Genort. Die Kartierung von chlD-Genen kann besonders für die Pflanzenzüchtung von Interesse sein.

chlD-spezifische Hybridisierungssonden oder CHLD-kodierende (Teil-)Sequenzen können auch dazu genutzt werden, um in planta die Expression eines chlD-Gens zu inhibieren (Antisense-Technologie).

Mittels Standard-Methoden können CHLD-kodierende (Teil-)Sequenzen auch verändert oder um neue Sequenzelemente erweitert und in das Genom von Pflanzen eingebracht werden. Die Pflanzen können dabei nach herkömmlichen Verfahren transient oder stabil transformiert werden. Die Integration von Sequenzen abgeleitet aus der chlD-cDNA in das Genom der Pflanze kann dazu genutzt werden, die Eigenschaften der Pflanze zu verändern, so daß z. B. mehr oder weniger funktionell aktive Mg-Chelatase oder eine Variante der Mg-Chelatase mit veränderten Eigenschaften in der transgenen Pflanze gebildet wird oder aber, daß das Expressionsniveau des in der transgenen Pflanze vorhandenen chlD-Gens vermindert wird. Beispielsweise kann die Menge an funktioneller Mg-Chelatase durch Standard-Methoden erhöht werden, wenn z.B. das chlD-Gen alleine oder zusammen mit Genen anderer Mg-Chelatase-Untereinheiten unter der transkriptionellen Kontrolle regulatorischer Elemente wie Promotoren und Enhancern in die transgene Pflanze kloniert wird. Eine Erhöhung der CHLD-Aktivität kann z. B. zur Steigerung des Chlorophyllgehaltes, welche mit einer Ertragssteigerung verbunden sein kann, oder zur Erhöhung der Toleranz gegenüber Herbiziden, welche die Chlorophyllbiosynthese inhibieren, eingesetzt werden. Ebenso kann z.B. die Klonierung von veränderten, erfindungsgemäßen Sequenzen in transgene Pflanzen mit einer Erhöhung der Herbizidtoleranz verbunden sein.

Die in transgene Pflanzen eingeführten Nukleinsäuremoleküle, kodierend für chlD-Sequenzen oder Fragmente aus diesen Sequenzen (Molekülen), können aber auch in einer Weise mit regulatorischen Elementen, wie beispielsweise Promotoren oder Enhancern, versehen werden, so daß eine Erniedrigung der ursprünglichen Mg-Chelatase-Aktivität in der transgenen Pflanze resultiert (z.B. Kosuppression, Bildung von chlD-Antisense-RNA). Eine Herabsetzung der Mg-Chelataseaktivität kann zur Herstellung von chlorotischen Pflanzen oder Pflanzen mit chlorotischen Geweben genutzt werden. Ein verminderter Chlorophyllgehalt kann beispielsweise bei einer späteren technischen Verarbeitung von Pflanzen oder Pflanzenteilen erwünscht sein oder bei der Züchtung verschiedener Nutz- oder Zierpflanzen, z. B. Gemüsesorten wie Chicorée.

Die vorliegende Erfindung betrifft außerdem ein Protein, dadurch gekennzeichnet, daß das Protein die biologische Funktion einer pflanzlichen Mg-Chelatase-Untereinheit CHLD aufweist, vorzugsweise gemäß SEQ ID Nr. 2, oder ein biologisch aktives Fragment daraus.

Gegenstand der Erfindung ist auch die Verwendung eines Proteins mit der biologischen Funktion einer pflanzlichen Mg-Chelatase-Untereinheit CHLD, vorzugsweise gemäß SEQ ID Nr. 2 oder eines biologisch aktiven Fragments daraus, zur Aktivitätsbestimmung einer Mg-Chelatase sowie zur Herstellung von Antikörpern.

Gegenstand der Erfindung sind auch Verfahren zur Herstellung eines Proteins mit der Funktion einer pflanzlichen Mg-Chelatase Untereinheit CHLD in einer rekombinanten Wirtszelle, dadurch gekennzeichnet, daß diese Wirtszelle mit einem erfindungsgemäßen Nukleinsäuremolekül kodierend für eine pflanzliche Mg-Chelatase Untereinheit CHLD oder ein aktives Fragment daraus transformiert wird, vorzugsweise eine DNA-Sequenz kodierend für ein Protein mit der Funktion einer Mg-Chelatase oder ein biologisch aktives Fragment daraus in eine für die Wirtszelle geeignete Expressionskassette inseriert wird, die so erhaltene Expressionskassette in geeigneter Weise in einen für die Wirtszelle geeigneten Vektor inseriert wird, eine geeignete Wirtszelle mit dem so erhaltenen Vektor transformiert wird und die so transformierte Wirtszelle in einem geeigneten Medium kultiviert wird und das von besagter Wirtszelle produzierte Protein mit der Funktion einer pflanzlichen Mg-Chelatase Untereinheit CHLD in geeigneter Weise aus dem Kulturmedium oder der Wirtszelle isoliert wird.

Außerdem ist noch ein Gegenstand der vorliegenden Erfindung ein Verfahren zur Herstellung eines Proteins mit der Funktion einer pflanzlichen Mg-Chelatase oder einem biologisch aktivem Fragment daraus in einer rekombinanten Wirtszelle, dadurch gekennzeichnet, daß diese Wirtszelle mit einem erfindungsgemäßen Nukleinsäuremolekül kodierend für eine pflanzliche Mg-Chelatase oder ein aktives Fragment daraus transformiert wird, vorzugsweise eine DNA-Sequenz kodierend für ein Protein mit der Funktion einer Mg-Chelatase in eine für die Wirtszelle, geeignete Expressionskassette inseriert wird; die so erhaltene Expressionskassette in geeigneter Weise in einen für die Wirtszelle geeigneten Vektor inseriert wird, eine geeignete Wirtszelle mit dem so erhaltenen Vektor transformiert wird; und die so transformierte Wirtszelle in einem geeigneten Medium kultiviert wird und das von besagter Wirtszelle produzierte Protein mit der Funktion einer pflanzlichen Mg-Chelatase in geeigneter Weise aus dem Kulturmedium oder der Wirtszelle isoliert wird.

Die vorlegende Erfindung betrifft insofern die Herstellung der pflanzlichen Mg-Chelatase Untereinheit CHLD sowie der pflanzlichen Mg-Chelatase auf gentechnischem Wege. Z. B. können zur Herstellung der erfindungsgemäßen Proteine in einem Wirtsorganismus die erfindungsgemäßen DNA-Sequenzen in eine Expressionskassette kloniert werden, welche zur heterologen Expression des Strukturgens in dem ausgewählten Wirtsorganismus geeignet ist.

Hierfür sind beispielsweise folgende CHLD-kodierenden DNA-Sequenzen geeignet: pflanzliche chlD-cDNA, mit gängigen Methoden in ihrer Sequenz veränderte pflanzliche chlD-cDNA-Moleküle, aber auch synthetische DNA-Sequenzen, abgeleitet aus pflanzlicher chlD-cDNA oder pflanzlichem CHLD-Protein oder deren Fragmente, die die Expression einer biologisch aktiven Mg-Chelatase CHLD ermöglichen. Die Einführung spezifischer regulatorischer Sequenzen in die Expressionskassette kann außerdem erwünscht sein, beispielsweise von Promotoren, Operatorsequenzen, Enhancern, Terminatoren, Signalsequenzen, 5'- und 3'-untranslatierte Sequenzen oder Sequenzen kodierend für geeignete Fusionsproteine. Die Verwendung regulatorischer Sequenzen ist allgemein übliche Technik, die je nach Expressionsstategie breit variieren kann. Die resultierende chlD-Expressionskassette, versehen mit den notwendigen regulatorischen Elementen im passenden Leserahmen des chlD-Strukturgens, kann in einen Expressionsvektor, mit welchem der ausgewählte Wirtsorganismus transformiert werden kann, inseriert werden. Geeignete Expressionsstrategien zur Herstellung rekombinanter Proteine und entsprechende Expressionsvektoren sind allgemein bekannt für Wirtsorganismen wie beispielsweise *Escherichia coli*, Insekten- und Pflanzenzellen.

Der Begriff "Vektor" bezeichnet im allgemeinen ein geeignetes, dem Fachmann bekanntes Vehikel, das den gezielten Transfer eines ein- oder doppelsträngigen Nukleinsäuremoleküls in eine Wirtszelle ermöglicht, beispielsweise einen DNA- oder RNA-Virus, ein Virusfragment, ein Plasmidkonstrukt, das in An- oder Abwesenheit von regulatorischen Elementen zum Nukleinsäure-Transfer in Zellen geeignet sein kann, Metallpartikel wie sie z.B. beim "particle gun"-Verfahren eingesetzt werden können, er kann aber auch ein Nukleinsäuremolekül umfassen, das durch chemische oder physikalische Verfahren direkt in eine Zelle gebracht werden kann.

Der durch stabile oder transiente Transformation mit z.B. einer chlD-Expressionskasette erhaltene rekombinante Organismus kann zur Gewinnung von rekombinanter Mg-Chelatase, vorzugsweise CHLD-Protein, oder von Zellfraktionen, enthaltend CHLD dienen. Der rekombinante Organismus kann jedoch auch direkt der Bestandteil eines analytischen Testsystems sein.

Das chlD-Strukturgen kann auch zusammen mit den Strukturgenen kodierend für die beiden anderen bekannten Untereinheiten der Mg-Chelatase, CHLH und CHLI, mit Hilfe gängiger molekulargenetischer Verfahren in einen Wirtsorganismus eingebracht werden, so daß es dort zur Expression einer funktionsfähigen Mg-Chelatase kommt.

Ein bevorzugtes Expressionssystem ist z. B. die Verwendung von Bäckerhefe (*Saccharomyces cerevisiae*) als Wirtsorganismus. Als Vektoren können alle bekannten Hefe-Vektoren dienen, die über die geeigneten Expressionssignale, wie Promotoren, und geeignete Selektionsmarker, wie Resistenzgene oder Gene, die eine Auxotrophie komplementieren, verfügen.

Die Verwendung der pflanzlichen Mg-Chelatase basiert im wesentlichen auf ihrer Aktivität als heterooligomerer Enzymkomplex, die unmittelbar an die Anwesenheit der Untereinheit CHLD gebunden ist. Die Bereitstellung funktionell intakter, pflanzlicher Mg-Chelatase ermöglicht sowohl in vitro (z.B. zellfreies Testsystem der Mg-Chelatase) als auch in vivo die Durchführung von biochemischen Reaktionen, beispielsweise in ein- oder mehrzelligen rekombinanten Organsimen oder Zellkulturen, insbesondere Hefen, Bakterien, Algen, Insektenzellen oder Pflanzen, und ist insofern nicht auf phototrophe Organismen beschränkt.

Diese Reaktionen können einerseits zur Herstellung von Mg-Tetrapyrrolen genutzt werden, andererseits können diese biochemischen Reaktionen dazu verwendet werden, um in einem Testsystem die Wirkung von chemischen Verbindungen, aber auch von heterogenen Stoffgemischen, in bezug auf die Funktion der Mg-Chelatase zu bestimmen.

Die vorliegende Erfindung betrifft daher auch noch ein Verfahren zur Bestimmung der Wechselwirkung pflanzlicher Mg-Chelatase Untereinheiten, dadurch gekennzeichnet, daß eine Wirtszelle mit einer DNA-Sequenz, kodierend für ein Protein mit Funktion einer CHLD-Untereinheit oder einem biologisch aktiven Fragment daraus, sowie mindestens mit einer DNA-Sequenz, kodierend für eine weitere Untereinheit der Mg-Chelatase, in einer Weise transformiert wird, daß die Interaktion der Mg-Chelatase-Genprodukte zu einem direkt oder indirekt, qualitativ oder quantitativ meßbaren Signal führt, vorzugsweise durch die Aktivierung eines Markergens.

Das Verfahren ist geeignet, spezifische Inhibitoren oder Aktivatoren der pflanzlichen Mg-Chelatase aufzufinden, so daß u.a. Stoffe identifiziert werden können, welche eine potentielle herbizide, wachstumshemmende oder -fördernde oder phytosanitäre Wirkung besitzen.

Das Prinzip dieser zellulären (in vivo) Testsysteme beruht darauf, daß ein rekombinanter Organismus, der mit Strukturgenen der pflanzlichen Mg-Chelatase CHLD transformiert worden ist, sowie eine oder beide weiteren Untereinheiten der pflanzlichen Mg-Chelatase funktionell exprimiert, eine essentielle Funktion einer oder mehrerer Untereinheiten in der Weise anzeigt, daß damit Stoffe gefunden werden können, welche diese Funktion beeinflussen.

Eine Funktion der Mg-Chelatase-Untereinheiten besteht darin, miteinander in Wechselwirkung zu treten. Diese Wechselwirkung führt zur Bildung eines Enzymkomplexes und stellt eine Voraussetzung für die Funktion der Mg-Chelatase als Enzym dar.

Die vorliegende Erfindung betrifft daher ein Testsystem, mit dem die Interaktion der Mg-Chelatase-Untereinheit CHLD mit einer oder zwei weiteren Untereinheiten des Enzyms in vivo nachgewiesen und quantitativ bestimmt werden kann.

Die Interaktion der Untereinheit CHLD mit der Untereinheit CHLI kann beispielsweise dadurch gemessen werden, daß beide Untereinheiten so mit zwei weiteren Polypeptiden oder Proteinen verbunden werden, daß in der Zelle durch die Interaktion der Untereinheiten direkt oder indirekt eine qualitativ und/oder quantitativ detektierbare Reaktion verursacht wird.

Hierfür geeignete Polypeptide sind z.B. Domänen oder Untereinheiten von regulatorischen Proteinen, beispielsweise aus der zellulären Signaltransduktion oder aus Transkriptionsvorgängen, deren Funktion über eine Protein-Protein-Wechselwirkung vermittelt wird.

Beispielsweise sind regulatorische Proteine der DNA-Transkription geeignet, bei denen die Protein-Protein-Wechselwirkung von zwei oder mehreren Untereinheiten mit Hilfe eines Reportergens nachgewiesen werden kann, so daß das Genprodukt des Reportergens nur dann gebildet wird, wenn zwei oder mehrere der o.g. Untereinheiten miteinander in Wechselwirkung treten.

Um ein solches Testsystem herzustellen, kann z.B. eine der beiden zu testenden Mg-Chelatase-Untereinheiten mit der DNA-Bindedomäne, die andere Mg-Chelatase-Untereinheit mit der transkriptionsaktivierenden Domäne des regulatorischen Proteins fusioniert werden. Dabei ist unerheblich, welches der beiden Proteine mit welcher der beiden Domänen verbunden wird. So kann CHLD mit der DNA-Bindedomäne und CHLI mit der transkriptionsaktivierenden Domäne verbunden werden. Diese Bindung kann über die Wechselwirkung eines weiteren Hilfsfaktors vermittelt werden, beispielsweise eine Protein-Protein-Wechselwirkung oder auch eine Protein-Ligand-Wechselwirkung, wie z. B. Streptavidin-Biotin. Bevorzugt wird jedoch eine kovalente Bindung, welche beispielweise durch Fusion von kodierenden Regionen betreffender Gene erfolgt.

Ein Beispiel für einen solches regulatorisches Protein ist das Genprodukt des GAL4-Gens aus Saccharomyces cerevisiae.

Ein Wirtsorganismus, in dessen Genom bereits ein Indikatorgen mit dem entsprechenden Promotor integriert wurde, wird mit den beschriebenen Fusionsgenen der Mg-Chelatase-Untereinheiten transformiert. Ein solcher Wirtsorganismus zeichnet sich dadurch aus, daß er alle Funktionen des verwendeten Transkriptionssystems exprimiert, ausgenommen dasjenige regulatorische Protein, welches als rekombinantes Protein exprimiert werden soll. Wird als Wirtsorganismus ein Mikroorganismus verwendet, so kann anschließend der rekombinante Organismus mit gängigen mikrobiologischen Methoden isoliert und beliebig vermehrt werden und steht damit in unbegrenztem Ausmaß für den Einsatz in dem Testsystem zur Verfügung. Bevorzugte Verwendung als Wirtsorganismus finden daher leicht transformier- und kultivierbare Mikroorganismen, wie beispielsweise Hefearten oder *E. coli* .

Des weiteren wird ein für das Transkriptionssystem geeigneter Promotor mit einem Strukturgen verbunden, welches für ein Indikatorprotein kodiert. Dieses rekombinante Gen zeichnet sich dadurch aus, daß die Aktivierung des Promotors mittelbar oder unmittelbar zur Expression des Indikatorproteins führt. Ein solches Indikatorprotein zeichnet sich dadurch aus, daß es in dem rekombinanten Organismus zu einer leicht detektierbaren Veränderung führt, indem es beispielsweise eine Farbstoffbildung oder eine Chemoluminiszenzreaktion katalysiert, selbst farbig ist oder fluoresziert, oder zum Wachstum des Mikroorganismus beiträgt.

Die Verwendung des dargestellten Systems der Trankriptionsaktivierung eines Indikatorgens hat gegenüber dem weiter unten beschriebenen Wachstumstest einer mit einer Mg-Chelatase-Untereinheit komplementierten Mutante den Vorteil, daß mit ersterem ein spezifischeres Signal auf einen Inhibitor des Enzyms bzw. einer Enzymfunktion möglich ist, während bei letzterem Test erst Inhibitoren des Zellwachstums mit anderem Angriffspunkt als der Mg-Chelatase durch Kontrollexperimente ausgeschlossen werden müssen. Die vorliegende Erfindung bevorzugt daher gegenüber dem Wachstumstest die Verwendung eines Indikatorgensystems.

Als Beispiel für einen solches System, das die oben genannten Wechselwirkungen mit Hilfe regulatorischer Proteine des Transkriptionssystems verwirklicht, sei insbesondere das Matchmaker™ two-hybrid-system der Firma Clontech, Palo Alto, California, USA, Katalog-Nr. #K 1605-1 aus dem Jahr 1995/96, genannt.

Hierfür werden Plasmide hergestellt, welche die Expression von CHL D, H und I als Fusionsproteine mit der GAL4-Bindedomäne und der GAL4-Aktivierungsdomäne in Hefezellen bewirken. Zur Konstruktion der Expressionsplasmide werden die DNA-Abschnitte, welche für die drei Untereinheiten der Mg-Chelatase kodieren, mittels Polymerase-Kettenreaktion (PCR) amplifiziert.

Zur Amplifizierung werden als Templates die Plasmide verwendet, welche die cDNA der jeweiligen Gene enthalten. Als PCR-Primer werden synthetische Oligonukleotide eingesetzt, die sich dadurch auszeichnen, daß der sense-Primer Homologie zum 5'-Ende, der antisense-Primer Homologie zum 3'-Ende des jeweiligen Gens aufweist. Die Primer können ferner mit Restriktionsschnittstellen versehen sein, die eine Klonierung in Vektorplasmide erleichtern.

Als Vektoren werden bevorzugt die Shuttle-Plasmide des MATCHMAKER™ Zwei-Hybrid-Systems (Clontech) verwendet: pGBT9 und pAS2 enhalten jeweils ein Gen, das für die GAL4-Aktivierungsdomäne kodiert pGAD424 und pACT2 enhalten jeweils ein Gen, das für die GAL4-DNA-Bindedomäne kodiert. pGBT9 und pGAD424, beziehungsweise pAS2 und pACT2, werden jeweils zusammen in einem System eingesetzt. pGBT9 und pGAD424 unterscheiden sich von pAS2 und pACT2 dadurch, daß bei ersteren die genannten Gene schwächer exprimiert werden als bei letzteren.

Die für die Mg-Chelatase kodierenden Untereinheiten werden jeweils einzeln in die Vektoren kloniert. Rekombinante Plasmide werden durch Transformation in E. coli eingebracht, vermehrt und isoliert. Auf diese Art werden alle 12 möglichen Kombinationen aller drei Untereinheiten mit allen vier Vektoren hergestellt. Die rekombinanten Plasmide werden wie folgt bezeichnet:

Gen CHL D in Vektor pAS2 wird bezeichnet als pCBS1148.
Gen CHL D in Vektor pACT2 wird bezeichnet als pCBS1149.
Gen CHL D in Vektor pGBT9 wird bezeichnet als pCBS1150.
Gen CHL D in Vektor pGAD424 wird bezeichnet als pCBS1151.
Gen CHL H in Vektor pAS2 wird bezeichnet als pCBS1152.
Gen CHL H in Vektor pACT2 wird bezeichnet als pCBS1153.
Gen CHL H in Vektor pGBT9 wird bezeichnet als pCBS1154.
Gen CHL H in Vektor pGAD424 wird bezeichnet als pCBS1155.

Gen CHL I in Vektor pAS2 wird bezeichnet als pCBS1156.
Gen CHL I in Vektor pACT2 wird bezeichnet als pCBS1157.
Gen CHL I in Vektor pGBT9 wird bezeichnet als pCBS1158.
Gen CHL I in Vektor pGAD424 wird bezeichnet als pCBS1159.

Anschließend wird ein Reporter-Hefestamm des Zweihybrid-Systems mit jeweils zwei Plasmiden transformiert. Als Reporter-Hefestamm wird bevorzugt ein Stamm verwendet, welcher als Indikatorprotein eine durch GAL4 induzierbare beta-Galaktosidase exprimiert, bevorzugt der Stamm SFY526 von Clontech. Die zwei Plasmide werden so gewählt, daß jeweils eines eine Untereinheit in Fusion mit der DNA-Bindedomäne, das andere eine Untereinheit in Fusion mit der Aktivierungsdomäne kodiert. Die Hefe-Transformation wird z.B. nach der Methode von Klebe et al., 1983, Gene, 25, 333-341 durchgeführt. Zur Bestimmung der Induktion des Indikatorgens wird eine mit beiden Plasmiden transformierte Hefe auf Festmedium angezogen und die Aktivität der beta-Galaktosidase wird nach Blotten auf Filter nach der Methode von Breedon und Nasmyth (Breedon, L. and Nasmyth, K. (1985) Regulation of the yeast HO gene. Cold Spring Harbor Symp. Quant. Biol. 50, 643-650) bestimmt.

Alternativ wird die transformierte Hefe in Flüssigmedium angezogen und die Aktivität der beta-Galaktosidase wird nach Zellaufschluß in Hefe-Rohextrakt nach der Methode von Munder und Fürst (Munder, T. and Fürst, P. (1992). Mol. Cell. Biol. 12, 2091-2099) bestimmt.

Zum Testen von Stoffen wird der rekombinante Organismus zunächst in einem geeigneten Medium angezogen. Anschließend werden die rekombinanten Zellen zusammen mit dem zu testenden Stoff inkubiert. Die Inkubationsbedingungen werden so gewählt, daß während der Inkubationszeit ohne Zusatz zu testender Stoffe eine deutlich meßbare Induktion des Indikatorgens stattfindet. Dies kann z. B. dadurch geschehen, daß sich die Inkubationszeit über einen Teil der Wachstumsphase erstreckt.

Alternativ kann die Induktion auch dadurch geschehen, daß die an der Transkriptionsaktivierung beteiligten Proteine mit Hilfe eines induzierbaren Promotors exprimiert werden. Die Induktion des Indikatorgens kann mit gängigen Detektionsreaktionen nachgewiesen werden. Wenn das Indikatortergen in dem Testsytem aktiviert wird, wird als Folge der Genaktivierung eine größere Menge des Reporterproteins in den Wirtszellen gebildet, als ohne Aktivierung des Indikatorgens. Bevorzugt wird das Testsystem so ausgewählt, daß das Reporterprotein mindestens doppelt so stark produziert wird. Besonders bevorzugt wird eine mindestens zehnfache Steigerung der Produktion des Reporterproteins erzielt. Sofern es sich bei dem Reporterprotein um ein Enzym handelt, kann die Enzymaktivität mittels gängiger Meßmethoden, beispielsweise colorimetrisch, fluorimetrisch oder luminometrisch, bestimmt werden. Dazu können die ganzen Zellen oder daraus gefertigte Extrakte verschiedenen Reinheitsgrades mit einem geeigneten farbbildenden Enzymsubstrat inkubiert werden. Die Inkubation mit Substrat kann bereits während der Inkubation mit dem zu testenden Stoff oder auch danach erfolgen.

Wenn eine chemische Verbindung diese Interaktion zwischen den Untereiheiten stört, indem sie z. B. an einer "Interfaceregion" einer Untereinheit bindet, hemmt diese Verbindung den Prozeß der Oligomerisierung. Somit kann diese Verbindung die Menge an enzymatisch aktiver Mg-Chelatase in einem Organismus vermindern und somit auch potentielle herbizide Wirkung besitzen. Über dieses Test-System können auf diese Weise auch herbizide Verbindungen gefunden werden, welche die Bildung des aktiven Mg-Chelatase Enzymkomplexes hemmen.

Um eine spezifische Interaktion eines Stoffes mit einem Protein der Mg-Chelatase nachzuweisen, muß der Stoff folgende Bedingungen erfüllen:

1. In rekombinanten Zellen muss nach Zusatz des Stoffes eine signifikant geringere Induktion des Indikatorgens stattfinden als in Zellen ohne Zusatz des Stoffes.

2. In einem analogen Transkriptionssystem (Kontrollsystem), bei dem die Wechselwirkung der Mg-Chelatase-Untereinheiten durch eine andere Wechselwirkung ersetzt wurde, darf nach Zusatz des Stoffes nur eine deutlich geringere oder keine meßbare Hemmung der Indikatorreaktion auftreten. Als andere Wechselwirkung kann jede Art der Bindung dienen, beispielsweise auch kovalente Verknüpfung von Proteinen.

Erfüllt ein Stoff die genannten Bedingungen, so kann er in weiteren Testsystemen auf seine Wirkung untersucht werden, beispielsweise in enzymatischen Tests mit der Mg-Chelatase, in Pflanzenzellkulturen oder in herbologischen Tests an ganzen Pflanzen.

Ein Mg-Chelatase-Gen kann außerdem in eine bestimmte Mutante eines solchen Organismus eingebracht werden, die ohne die Funktion der entsprechenden Mg-Chelatase nicht wachstumsfähig ist, wie z. B. ein photoautotropher Mikroorganismus, bei dem eine oder mehrere Untereinheiten der Mg-Chelatase ihre Funktion durch eine Mutation verloren haben. Die Verwendung einer solchen Mutante hat den besonderen Vorteil, daß dadurch das Wachstum des rekombinanten Organismus in einem geeignetem Medium als Maß für die Funktion der Mg-Chelatase gelten kann und somit den Einfluß von zu untersuchenden Stoffen auf die Mg-Chelatase quantitativ beschreiben kann. Der Wachstumstest zeichnet sich durch eine besonders einfache Handhabung und den schnellen Durchsatz von Stoffen aus.

Dazu kann das pflanzliche chlD-Gen in eine Mutante eines Organismus gebracht werden, die dadurch gekennzeichnet ist, daß sie ohne die Funktion der Mg-Chelatase, bevorzugt ohne die Funktion des CHLD-Proteins, unter bestimmten Kulturbedingungen nicht wachstumsfähig ist oder einen veränderten Phänotyp aufweist. Eine solche Mutante kann beispielsweise hergestellt werden, indem bei einem Mikroorganismus, der zum phototrophen Wachstum die Aktivität einer Mg-Chelatase benötigt, zunächst die Strukturgene für seine eigene Mg-Chelatase derart verändert werden, daß kein oder keine ausreichenden Mengen funktionsfähiges Enzym mit Mg-Chelataseaktivität mehr gebildet werden, so daß der resultierende Organismus nicht mehr phototroph wachsen kann. In dieser Mutante werden des weiteren die pflanzlichen Strukturgene chlI und chlH zur Expresion gebracht. Durch zusätzliche Expression des pflanzlichen chlD-Strukturgens kann dann in dem gentechnisch veränderten Organismus eine funktionelle pflanzliche Mg-Chelatase gebildet werden, die diesem Organismus die Fähigkeit zu phototrophem Wachstum wieder verleiht. Das Wachstum eines solchen rekombinanten Organismus stellt dann direkt ein Maß für die Aktivität der pflanzlichen Mg-Chelatase dar. Durch einen Wachstumstest kann mit diesem rekombinanten Organismus bestimmt werden, ob die pflanzliche Mg-Chelatase durch eine chemische Verbindung, welche dem Kulturmedium zugesetzt wird, inhibiert wird. Dazu wird der gentechnisch veränderte Organismus in Kulturmedien mit und ohne die zu untersuchende Verbindung unter phototrophen Kulturbedingungen angezogen. Die zu untersuchende chemische Verbindung wird dabei bevorzugt in Konzentrationen zwischen

$10^{-9}$ M und $10^{-3}$ M, und besonders bevorzugt in Konzentrationen zwischen $10^{-7}$ M und $10^{-4}$ M eingesetzt.

Um eine spezifische Inhibition der Mg-Chelatase durch eine zu untersuchende chemische Verbindung nachzuweisen und andere Wirkungsweisen als Ursache für eine Wachstumshemmung auszuschließen, muß die Verbindung folgende Kriterien erfüllen:

1. Die gentechnisch veränderten Organismen müssen in Gegenwart der Verbindung signifikant schlechter wachsen als in Kulturmedium ohne die Verbindung. 2. Die Verbindung darf das Wachstum der gleichen Organismen unter heterotrophen Bedingungen nicht signifikant vermindern.

In gleicher Weise wie das Wachstum, kann auch ein veränderter Phänotyp eines entsprechend veränderten Organismus als Indikator für die katalytische Aktivität der pflanzlichen Mg-Chelatase genutzt werden. Verschiedene Pigmente phototropher Mikroorganismen werden ausgehend von dem Reaktionsprodukt der Mg-Chelatase, Mg-Protoporphyrin, gebildet. Ein wie oben beschrieben konstruierter rekombinanter Mikroorganismus, der anstelle seiner eigenen Mg-Chelatase eine rekombinante pflanzliche Mg-Chelatase, enthaltend das rekombinante CHLD-Protein, besitzt, kann nur dann seine von Mg-Protoporphyrin abgeleiteten Pigmente bilden, wenn die pflanzliche Mg-Chelatase eine ausreichende Aktivität aufweist. Die Pigmentierung eines solchen rekombinanten Organismus stellt dann direkt ein Maß für die Aktivität der pflanzlichen Mg-Chelatase dar. Durch Analyse der Pigmentzusammensetzung kann mit diesem rekombinanten Organismus bestimmt werden, ob die pflanzliche Mg-Chelatase durch eine chemische Verbindung, welche dem Kulturmedium zugesetzt wird, inhibiert wird. Dazu wird der gentechnisch veränderte Organismus in Kulturmedien mit und ohne der zu untersuchenden Verbindung unter solchen Kulturbedingungen angezogen, unter denen aus Mg-Protoporphyrin synthetisierte Pigmente gebildet werden. Die zu untersuchende chemische Verbindung wird dabei bevorzugt in Konzentrationen zwischen $10^{-9}$ M und $10^{-3}$ M, und besonders bevorzugt in Konzentrationen zwischen $10^{-7}$ M und $10^{-4}$ M eingesetzt. Um eine spezifische Inhibition der Mg-Chelatase durch eine zu untersuchende chemische Verbindung nachzuweisen und andere Wirkungsweisen als Ursache für eine Veränderung in der Pigmentierung auszuschließen, muß die Verbindung folgende Kriterien erfüllen:

1. Die gentechnisch veränderten Organismen weisen in Gegenwart der Verbindung signifikant geringere Mengen an Pigmenten, die ausgehend von Mg-Protoporphyrin gebildet werden, oder eine veränderte Zusammensetzung an Pigmenten auf, als in Kulturmedium ohne die Verbindung.

2. Die Verbindung darf die Pigmentbildung des nicht gentechnisch veränderten Ausgangsorganismus unter den gleichen Kulturbedingungen nicht signifikant verändern.

Wenn beide Kriterien erfüllt sind, ist davon auszugehen, daß die untersuchte Verbindung ein spezifischer Inhibitor der pflanzlichen Mg-Chelatase ist. Wenn nur Bedingung 1 erfüllt ist, nicht aber Bedingung 2, kann mit Hilfe einer enzymatischen Reaktion der Mg-Chelatase festgestellt werden, ob die untersuchte Verbindung ein spezifischer Inhibitor der pflanzlichen Mg-Chelatase ist.

Erfüllt ein Stoff die obengenannten Bedingungen, so kann er mit oder ohne weitere biochemische Untersuchungen direkt an ganzen Pflanzen oder geeigneten Pflanzenteilen auf seine Wirkung in der Pflanze untersucht werden. Dazu können die gängigen herbologischen und physiologischen Verfahren angewandt werden. Für verschiedene Anwendungen wie z. B. die Herstellung eines biochemischen Testsystems zur Bestimmung einer Proteinfunktion ist eine wesentliche Voraussetzung, daß das zu untersuchende Protein in funktionsfähigem Zustand und möglichst rein, d. h. frei von störenden Aktivitäten, gewonnen werden kann. Wie bei allen Zellproteinen kann dies im Falle einer pflanzlichen Mg-Chelatase dadurch geschehen, daß die einzelnen Untereinheiten mit Hilfe gängiger Verfahren der Proteinreinigung aus pflanzlichem Gewebe isoliert werden. In der vorliegenden Erfindung ist dargelegt, daß eine funktionelle pflanzliche Mg-Chelatase neben den Untereinheiten CHLH und CHLI auch die Untereinheit CHLD enthält, deren Sequenz beispielhaft für *Nicotiana tabacum* in SEQ ID Nr. 2 angegeben ist. Generell ist die Isolierung eines heterooligomeren Proteins, dessen Aktivität zudem wie bei der Mg-Chelatase auch an eine Membranfraktion der pflanzlichen Zelle gebunden ist, wesentlich schwieriger als die eines löslichen oder homooligomeren beziehungsweise monomeren Enzyms, da das Protein im Verlauf der Reinigung seine enzymatische Aktivität verliert, die anschließend nur mit aufwendigen Methoden wiederhergestellt werden kann. Hinzu kommt, daß die Enzyme der Chlorophyllbiosynthese abhängig von der Art der Differenzierung und vom Entwicklungszustand der Zelle gebildet werden. Des weiteren stellen diese Proteine, wie auch die Mg-Chelatase, nur einen geringen Anteil am Gesamtprotein der Zelle dar, so daß bei einer Isolierung aus pflanzlichem Gewebe ausgesprochen hohe Anreicherungsfaktoren erreicht werden müßten.

Aufgrund der beschriebenen Schwierigkeiten wird gemäß der vorliegenden Erfindung die Verwendung der für die Mg-Chelatase kodierenden Nukleinsäuremoleküle zur Herstellung der rekombinanten pflanzlichen Mg-Chelatase bevorzugt, besonders bevorzugt des rekombinanten Mg-Chelatase Komplexes bestehend aus den Proteinen CHLD, CHLH und CHLI. Ganz besonders bevorzugt genannt sei das rekombinante pflanzliche CHLD-Protein.

Gentechnisch produzierte pflanzliche Mg-Chelatase, vorzugsweise gentechnisch hergestelltes CHLD-Protein, kann mittels einer Vielzahl von Standard-Methoden gereinigt werden. Die Eignung einer Methode hängt jeweils vom verwendeten Wirtsorganismus, der Expressionsstategie und anderen Faktoren ab, die einem in der Expression und Reinigung rekombinanter Proteine erfahrenen Fachmann bekannt sind. Zum Zweck der Reinigung kann das rekombinante Protein auch durch entsprechende Veränderung seiner Gen-Sequenz in der Expressionskassette mit weiteren Peptidsequenzen fusioniert werden. Bevorzugt sind als Fusionspartner Peptide oder Proteine zu verwenden, die als C- oder N-terminale Fusionen dem rekombinanten Mg-Chelatase Untereinheiten eine Affinität zu bestimmten Säulenmaterialien verleihen oder eine bestimmte Lokalisierung des Expressionsprodukts innerhalb oder außerhalb der Zelle ermöglichen, wie z.B. durch Transit- oder Signalpeptide oder -sequenzen. Solche Fusionen dürfen die Funktion der Mg-Chelatase nicht beeinflussen oder müssen z. B. durch Einbau geeigneter Proteaseschnittstellen abspaltbar sein. Als Beispiele für Fusionspartner seien Oligohistidin-Tails, das Strep-Tag™, die Glutathion-S-Transferase (GST) oder das Maltose-Bindende Protein (MalE) genannt, ohne daß diese Anwendung auf die beispielhaft angegebenen Fusionspartner oder deren Fragmente beschränkt ist. Für die Reinigung eines aktiven Mg-Chelatase Komplexes kann es auch ausreichend sein, wenn lediglich eine der im Komplex vorhandenen Untereinheiten mit einem derartigen Peptid oder Protein fusioniert ist. Des weiteren können ausgehend von bereits geringen Mengen rekombinant hergestellter Mg-Chelatase, bevorzugt des CHLD-Proteins, auch spezifische Antikörper hergestellt werden, mit deren Hilfe Mg-Chelatase oder Mg-Chelatase-Untereinheiten aus rekombinanten Organismen oder auch aus Pflanzen isoliert werden können.

Die gentechnische Herstellung der Mg-Chelatase, vorzugsweise des CHLD-Proteins, und deren Reinigung ermöglichen erstmalig, ein pflanzliches Enzym mit Mg-Chelatase-Aktivität in verschiedenen Reinheitsgraden bis hin zum reinen Mg-Chelatase-Komplex, bestehend aus den Untereinheite CHLD, CHLH und CHLI, oder auch das reine CHLD-Protein zu erhalten. Eine Anwendungsmöglichkeit für isolierte oder angereicherte Mg-Chelatase besteht z.B. in deren Verwendung in biochemischen Testsystemen zur Bestimmung der Enzymfunktion.

Die vorliegende Erfindung betrifft außerdem ein Verfahren zur Bestimmung der Aktivität pflanzlicher Mg-Chelatase, wobei ein Protein mit der Funktion einer CHLD mit den Genprodukten der DNA-Sequenzen kodierend für die Mg-Chelatase-Untereinheiten I und H in einer Weise zusammen gebracht wird, daß die enzymatische Aktivität der Mg-Chelatase-Genprodukte zu einem direkt oder indirekt, qualitativ oder quantitativ meßbaren Signal führt.

Der inhibitorische Effekt einer chemischen Verbindung auf die Mg-Chelatase kann durch eine Reduktion der typischen Katalyseaktivität des Enzyms oder der kompletten Inaktivierung der Mg-Chelatase in Gegenwart dieser Verbindung gemessen werden. Dazu wird die pflanzliche Mg-Chelatase, bestehend aus den Untereinheiten CHLD, CHLH und CHLI, in einem geeigneten Reaktionspuffer mit ihren Substraten Protoporphyrin, ATP und $Mg^{2+}$ inkubiert.

Als bevorzugte Reaktionsbedingungen seien ein Reaktionspuffer mit einem pH-Wert zwischen pH 4 und pH 11, vorzugsweise 5-10, insbesondere 6-9, und Reaktionstemperaturen zwischen 2 und 50°C, vorzugsweise 10-40°C genannt.

Die Quantifizierung der Enzyminhibition kann durch einen einfachen Vergleich der katalytischen Aktivität der Mg-Chelatase in Abwesenheit und in Anwesenheit der zu untersuchenden chemischen Verbindung geschehen.

Zur Bestimmung der Aktivität der Mg-Chelatase können verschiedene biochemische Meß-Methoden eingesetzt werden, durch die entweder die Entstehung der Reaktionsprodukte der von der Mg-Chelatase katalysierten Reaktion, z.B. Mg-Protoporphyrin, ADP oder Phosphat, gemessen wird oder aber die Abnahme der Konzentration der Enzymsubstrate der Mg-Chelatase, z.B. Protoporphyrin, ATP oder $Mg^{2+}$, gemessen wird. Dem in der Durchführung von Enzymtests erfahrenen Fachmann stehen viele Standardmethoden zur Bestimmung der Aktivität von Mg-Chelatase zur Verfügung. Bevorzugt genannt seien die Methoden zur Messung des Umsatzes von Protoporphyrin zu Mg-Protoporphyrin im Reaktionsansatz durch Messung der unterschiedlichen Fluoreszenseigenschaften von Mg-Protoporphyrin und Protoporphyrin (z. B. Walker et al., Plant Physiol. Biochem. 30:263-269 (1992)) oder durch photometrische Analyse der aus der Umsetzung von Protoporpyrin zu Mg-Protoporphyrin resultierenden Absorptionsänderung (z. B. Gorchein, Biochem. J. 299:869-874 (1991)) oder durch Quantifizierung von Protoporphyrin oder Mg-Protoporphyrin durch HPLC-Analyse. Als weitere bevorzugte Meßmethode sei die Bestimmung der ATPase-Aktivität der Mg-Chelatase genannt, wobei beispielsweise die Entstehung von Phosphat durch einen Phosphatnachweis, z. B. nach den Methoden basierend auf dem System beschrieben von Fiske et al., J. Biol. Chem 66:375-400 (1925), nachgewiesen werden kann. Darüber hinaus kann durch Variation der Konzentrationen des Substrates und des inhibierenden Stoffes mit gängigen biochemischen Verfahren der Hemm-Typ einer chemischen Verbindung an der pflanzlichen Mg-Chelatase bestimmt werden.

Anstelle von gereinigter Mg-Chelatase können auch ganze Zellen des rekombinanten Organismus, welcher die drei Untereinheiten CHLD, CHLH und CHLI der Mg-Chelatase rekombinant exprimiert, oder proteinhaltige Extrakte aus diesem Organismus oder verschieden stark angereicherte Mg-Chelatase-haltige Fraktionen aus diesem Organismus verwendet werden. Als bevorzugter rekombinanter Wirtsorganismus zu diesem Zwecke seien Hefezellen genannt. Alternativ kann auch eine aus Pflanzengewebe oder Pflanzenzellkulturen isolierte funktionsfähige Mg-Chelatase, enthaltend die Untereinheiten CHLD, CHLH und CHLI, verwendet werden.

Wird mit Hilfe der biochemischen oder zellbiologischen Testsysteme festgestellt, daß eine chemische Verbindung *in vitro* die pflanzliche Mg-Chelatase inhibiert, so kann diese Verbindung direkt an ganzen Pflanzen oder geeigneten Pflanzenteilen bezüglich ihrer Wirkung auf Pflanzen untersucht werden. Dazu stehen dem in der Bewertung von Wirkstoffen an Pflanzen erfahrenen Fachmann eine Vielzahl gängiger herbologischer und physiologischer Verfahren zur Verfügung.

Des weiteren kann die gentechnisch hergestellte Mg-Chelatase, vorzugsweise ein Mg-Chelatase-Komplex mit der gentechnisch hergestellten Untereinheit CHLD oder auch die freie CHLD-Untereinheit, beispielsweise auch verwendet werden, um die Raumstruktur des pflanzlichen Enzyms aufzuklären. Zur Aufklärung der Raumstruktur können allgemein bekannte Verfahren, wie z. B. die Röntgenstrukturanalyse von Proteinkristallen oder NMR-Spektroskopie verwendet werden. Die Strukturinformationen über die Mg-Chelatase, vorzugsweise die CHLD-Untereinheit können beispielsweise verwendet werden, um ein rationales Design von neuen Inhibitoren der Mg-Chelatase - und somit potentiellen Herbiziden - durchzuführen.

Auf die Erfindung der deutschen Patentanmeldung 197 17 656.9, deren Priorität die vorliegende Anmeldung beansprucht, sowie der Zusammenfassung der vorliegenden Anmeldung wird hiermit ausdrücklich Bezug genommen.

Die folgenden Beispiele dienen der näheren Erläuterung der Erfindung und stellen in keiner Weise eine Einschränkung dar:

Standard Methoden, wie DNA- und RNA-Isolierung, Sequenzanalyse, Restriktion, Klonierung, Gelelektrophorese, radioaktive Markierung, Southern- und Northem-Blot wurden nach gängigen Verfahren durchgeführt (Sambrook at al., 1989 Molecular Screening: A laboratory manual 2. Aufl. Cold Spring Harbour Laboratory Press, N.Y. USA; Sanger et al., 1977, Proc. Natl. Acad. Sci. USA 74, 5463-5467).

Beispiel 1: Klonierung einer Tabak-cDNA, die für die CHLD-Untereinheit der Mg-Chelatase kodiert

Zur Identifizierung der chlD-cDNA aus Tabak wurden die Peptidsequenzen VDASGS (SEQ ID Nr. 3), TDGRGN (SEQ ID Nr. 4) und AKGAVM (Seq. ID Nr. 7) ausgewählt, welche den aus dem chlD-Gen von Synechocystis PCC6803 (Jensen et al. (1996) J. Biol. Chem. 271, 16662-16667) abgeleiteten Proteinsequenzen entsprechen, um folgende Mischung aus DNA-Primersequenzen herzustellen:

1. : GA(CT) GT(ACTG) GA(GA) AA(AG) (TA)C(ACGT) GT(ACGT)

2.: AT (AG)TT (ACGT)CC (ACGT)CG (ACGT)CC (AG)TC (ACGT)G

3.: GC(ACGT) AA (AG) GG (ACGT) GC (ACGT) GT (ACGT) ATG C

Diese Primer wurden in einer Polymerase-Kettenreaktion (PCR) eingesetzt, um die genomische DNA aus Synechocystis PCC 6803 zu amplifizieren: 2 Zyklen mit jeweils 1 min 94°, 2 min 45°C, 3 min 72°C, dann 28 Zyklen jeweils 30 sec 94°C, 90 sec 60°C, 2 min 72°C.

Die PCR ergab ein DNA-Fragment von ca. 270 bp, das in den Klonierungsvektor pCRTMII (Invitrogen, San Diego) kloniert und zur weiteren Handhabung durch Restriktionsverdau isoliert wurde.

Mit dem isolierten und $^{32}$P[dCTP] markierten ca. 270 bp langen Fragment als Hybridisierungsprobe wurde eine Lambda ZAP II-cDNA-Bank von Tabak (Nicotiana tabacum SR1, Stratagene) nach Vorschrift gescreent.

Die nach cDNA-Bank-Screening im Phagemid p Bluescript SK- vorliegende DNA wurde isoliert und sequenziert. Die identifizierte und in SEQ ID Nr. 1 dargestellte chID-cDNA-Sequenz enthält einen 2274 Nukleotide langen offenen Leserahmen, sowie am 5'- und 3'-Ende nicht translatierte Regionen.

Genomische DNA von Tabak wurde mit radioaktiv markierter DNA des CHLD-Gens hybridisiert. Dazu wurde das CHLD-Gen mit EcoRI und HindIII aus dem Phagemid p-Bluescript SK- ausgeschnitten. Die resultierenden zwei Fragmente der Größen 2069 bp und 426 bp wurden mit $^{32}$P(dCTP) radioaktiv markiert.

Beispiel 2 Herstellung von chID-antisense Tabakpflan

Das gemäß Beispiel 1 in pBluescript SK- erhaltene Tabak-cDNA Fragment für CHLD wurde mit KpnI und XbaI aus dem Vektor pBluescript SK- herausgeschnitten und in den mit KpnI und XbaI aufgeschnittenen binären Vektor BinAR (Höfgen und Willmitzer, Plant Science (1990) 66, 22-230) hineinligiert.
Zunächst wurde der E. coli Stamm DH 5 a mit dem Konstrukt transformiert, anschließend Agrobacterium tumefaciens Stamm GV 2260. Mit der Blattscheiben Transformationsmethode (Horsch et al., Science 228, 1229-31) wurden junge Tabakblattscheiben mit den Agrobakterien inkubiert und das CHLD-antisense Gen in das pflanzliche Genom stabil integriert.

Beispiel 3 Herstellung von chID-sense Tabakpflanzen

Zur Herstellung der chID-sense Tabakpflanzen wurde die chID cDNA mit SmaI and XbaI aus p Bluescript SK- herausgeschnitten und in den BinAR Vektor nach Restriktionverdau hineinkloniert. Für die stabile Integration in das Tabakgenom wurde das in Beispiel 2 beschriebene Verfahren gewählt.

Beispiel 4 Analyse der transgenen Tabakpflanzen

Es wurden 67 Sense-Pflanzen und 56 Antisense-Pflanzen isoliert. Die transgenen Pflanzen mit Antisense- oder Sense-Genen für CHLD zeigten graduell unterschiedliche Chlorophylldefizienzen. Chlorotische Blätter wiesen diverse Variationsmuster auf. Es gab einheitlich entfärbte, gelblich-grüne Blätter, Blätter mit Flecken unterschiedlicher Pigmentierung, mit weißen Arealen entlang der Blattadern und grünen Interkostalfeldern. Mit zunehmenden Alter verloren die Blätter an Chlorophyll. Die Pflanzen mit reduziertem Chlorophyllgehalt zeigten auch einen beeinträchtigten Wuchs (siehe Fig. 1a bis 1d).

Des weiteren wurde die stabile Integration der Sense- oder Antisense-chID Gene in das Genom der Tabakpflanzen durch Southern-Blot Analyse bestätigt und durch Northern-Blot Analyse der mRNA-Gehalt des chID Transkripts bestimmt. In den Antisense-Pflanzen ist der Gehalt im Vergleich zum Wildtyp reduziert. chID-sense Pflanzen weisen im Vergleich zum Wildtyp nur leicht erhöhte chID mRNA Gehalte auf.

Die Aktivität der Mg-Chelatase wurde für transgene- und Wildtyppflanzen nach Lee et al. (1992, Plant Physiol. 99, 1134-1140) bestimmt. Die Sense- und Antisense-Pflanzen haben stets verringerte Enzymaktivitäten. Das Phänomen der reduzierten Enzymaktivitäten in den vermutlich CHLD-überexprimierten Transformanten kann mit Wirkung der negativen Dominanz erklärt werden. Ein Überangebot der CHLD-Untereinheit sorgt für eine Störung des fein regulierten Zusammenbaus des Mg Chelatase-Enzymkomplexes.

Der Gehalt an Protoporphyrin IX und Chlorophyll wurde in transgenen- und Wildtyppflanzen bestimmt (siehe Tabelle 2). Hervorzuheben ist, daß die Transformanten mit Sense- oder Antisense CHLD mRNA in ihren untersuchten jungen Blättern (3., 5. und 7. Blatt) in der Regel bis zu 3 - 5 fach erhöhte Protoporphyringehalte im Vergleich zu Wildtyppflanzen aufweisen.

Die Chlorophyllgehaltsbestimmung bestätigt den makroskopischen Phänotyp. Der Gehalt war in einzelnen Pflanzen bis zu 25 % reduziert.

Tabelle 2

| Relativer Protoprophyrin IX- und Chlorophyllgehalt in Sense- und Antisensepflanzen | | | |
|---|---|---|---|
| Pflanze | Blatt | Protoporphyrin IX [%] | Chlorophyll [%] |
| SNN | 3 | 100 | 100 |
| | 5 | 100 | |
| | 7 | 100 | 100 |
| AS7 | 3 | 584 | 25 |
| | 5 | 542 | |
| | 7 | 157 | 28 |
| AS9 | 3 | 135 | 50 |
| | 5 | 120 | - |
| | 7 | - | 49 |
| AS13 | 3 | 160 | 78 |
| | 5 | 102 | - |
| | 7 | 115 | 78 |
| AS18 | 3 | 374 | 74 |
| | 5 | 470 | - |
| | 7 | 295 | 80 |
| AS21 | 3 | 231 | 95 |
| | 5 | 188 | - |
| | 7 | 98 | 101 |
| S1 | 3 | 189 | 78 |
| | 5 | 262 | - |
| | 7 | 297 | 80 |
| S20 | 3 | 423 | 32 |
| | 5 | 293 | - |
| | 7 | 144 | 25 |
| S22 | 3 | 189 | 117 |
| | 5 | 151 | - |
| | 7 | 115 | 94 |
| S29 | 3 | 298 | 25 |
| | 5 | 265 | - |
| | 7 | 137 | 26 |
| S38 | 3 | 257 | 120 |
| | 5 | 127 | - |
| | 7 | - | 107 |
| SNN = Wildtyp<br>S = Sense<br>AS = Antisense | | | |

Beispiel 5: Konstruktion von Plasmiden für die Expression von CHL D, H und I als Fusionsproteine mit der GAL4-Bindedomäne und der GAL4-Aktivierungsdomäne

Zur Konstruktion der Expressionsplasmide wurde die DNA, welche für die drei Untereinheiten der Mg-Chelatase kodiert, mittels Polymerase-Kettenreaktion (PCR) amplifiziert.
Zur Amplifizierung des chlD-Gens wurden als Template 100 ng des Plasmids pNTCHLD verwendet. Als PCR-Primer wurden folgende beiden Oligonukleotide eingesetzt: 5'-TGA CCC GGG GGT AGT GGA ACC TGA AAA ACA ACC-3' als sense-Primer mit Smal-Restriktionsschnittstelle und als antisense-Primer entweder 5'-GGC GAA TTC TCA AGA TTC CTT TAA TGC AGA TAA-3' mit EcoRI-Restriktionsschnittstelle, oder 5'-GCG GTC GAC TCA AGA TTC CTT TAA TGC AGA -3' mit Sall-Restriktionsschnittstelle.

Zur Amplifizierung des CHLH-Gens wurden als Template 100 ng des Plasmids pNTCHLH verwendet. Als PCR-Primer wurden folgende beiden Oligonukleotide eingesetzt: als sense-Primer 5'-GCT GAT ATC GGC TAT TGG CAA TGG TTT ATT CAC-3' mit EcoRV-Restriktionsschnittstelle und als antisense-Primer 5'-GCG TCG ACA TTT ATC GAT CGA TTC CCT CAA-3' mit Sall-Restriktionsschnittstelle. Zur Amplifizierung des chll-Gens wurden als Template 100 ng des Plasmids pNTCHLI verwendet. Als PCR-Primer wurden folgende beiden Oligonukleotide eingesetzt: als sense-Primer 5'-CAG CCC GGG GGG TCC ACT ACT AGG-3' mit Smal-Restriktionsschnittstelle und als antisense-Primer 5'-CAG GTC GAG GCA CAG TAC AAA GCC-3' mit Sall-Restriktionsschnittstelle.
Als Vektoren wurden die Shuttle-Plasmide des MATCHMAKER™ Zwei-Hybrid-Systems (Clontech) verwendet: pGBT9 und pAS2 enhalten jeweils ein Gen, das für die GAL4-Aktivierungsdomäne kodiert. pGAD424 und pACT2 enhalten jeweils ein Gen, das für die GAL4-DNA-Bindedomäne kodiert. pGBT9 und pGAD424, beziehungsweise pAS2 und pACT2, werden jeweils zusammen in einem System eingesetzt. pGBT9 und pGAD424 unterscheiden sich von pAS2 und pACT2 dadurch, daß bei ersteren die genannten Gene schwächer exprimiert werden als bei letzteren.

Zur Klonierung von chlD in Vektor pACT2 wurde das Gen mittels des sense-Primers mit SmaI-Schnittstelle und des antisense-Primers mit EcoRI-Schnittstelle amplifiziert und anschließend mit dem mit Smal und EcoRI geschnittenen Vektor ligiert.

Zur Klonierung von chlD in jeweils einen der Vektoren pAS2, pGBT9 und pGAD424 wurde das Gen mittels des sense-Primers mit Smal-Schnittstelle und des antisense-Primers mit SalI-Schnittstelle amplifiziert und anschließen d mit dem mit SmaI und SalI geschnittenen Vektor ligiert.

Zur Klonierung von jeweils einem der Gene chlH und I in jeweils einen der Vektoren pAS2, pGBT9 und pGAD424 wurde die Gene jeweils mittels des sense- und des antisense-Primers amplifiziert und anschließend mit den jeweils mit Smal und SalI geschnittenen Vektoren ligiert.

Zur Klonierung von jeweils einem der Gene chlH und I in den Vektor pACT2 wurden die Gene mittels des sense- und des antisense-Primers amplifiziert und anschließend mit dem mit Smal und Xhol geschnittenen Vektor ligiert. Die ligierte DNA wurde mittels Transformation in E. coli DH 5a eingebracht und transformierte Klone wurden auf Agar-Medium mit 100 µg Ampicillin/ml selektiert. Aus den rekombinanten Bakterien wurde Plasmid-DNA isoliert und mittels Restriktionsanalyse auf ihre Identität überprüft.

Auf diese Art wurden alle 12 möglichen Kombinationen aller drei Untereinheiten mit allen vier Vektoren hergestellt.

Beispiel 6: Bestimmung der Interaktion zwischen Untereinheiten der Mg-Chelatase mittels des Zwei-Hybrid-Systems in Hefe

β-Galactosidase-Flüssigassay in Hefe (Munder und Fürst, 1992, Mol.Cell.Biol. 12, 2091-2099) am Beispiel des Stammes SFY526+pAS2-chlD+pACT2-chlI

Es wurden kompetente Hefezellen, Stamm SFY 526 (Harper et al., 1993, Cell, 75, 805-816), nach der Methode von Klebe hergestellt (Klebe et al., 1983, Gene, 25, 333-341) und mit den unter Beispiel 2 aufgeführten Plasmiden transformiert. Dabei wurde SFY 526 mit allen Plasmiden einzeln, als auch in allen möglichen Kombinationen transformiert und auf Minimalagar (1,5% Agar, 10% YNB-Glukose) unter Zusatz von Aminosäuren (Adenin 20mg/l; L-Histidin-HCl 20mg/l; L-Lysin-HCl 30mg/l; L-Leucin 30mg/l L-Tryptophan 20mg/l) selektiert.

Der zu vermessende Hefestamm SFY526+pAS2-chlD+pACT2-chlI wurde in 10ml Minimalmedium (10% YNB-Glukose) unter Zusatz benötigter Aminosäuren (Adenin 20mg/l; L-Histidin-HCL 20mg/l und L-Lysin-HCL 30mg/l) über Nacht bei 30°C und 180 rpm in einer sterilen 50ml-Steilbrustflasche mit Metallkappe kultiviert. Die optische Dichte ($OD_{600}$) dieser Übernachtkultur wurde erfaßt (Beckman DU 640 Spectrophotometer), die gemessenen Werte lagen dabei zwischen 1,0 und 2,0. 100µl von dieser Kultur wurden auf β-Galactosidase-Aktivität getestet. Das Probenvolumen betrug insgesamt ca. 1ml:

100 µl Kultur
700 µl Z-Puffer + Mercaptoethanol
50 µl Trichlormethan
50 µl 0,1% SDS

Parallel wurde ein Leerwert mitgeführt:

700 µl Z-Puffer
50 µl Trichlormethan
50 µl 0,1% SDS

Probe und der mitgeführte Leerwert wurden für jeweils 30 Sekunden gevortext und je 160µl ONPG-Lösung (4mg o-Nitrophenyl β-$_D$-Galactopyranoside auf 1ml Z-Puffer + Mercaptoethanol) zugegeben. Danach wurden die Proben vorsichtig geschüttelt und bei 30°C im Wasserbad inkubiert. Nach einer Stunde wurde die Reaktion durch Zugabe von 400µl 1 M $Na_2CO_3$ abgestopt und die Proben 10 min bei 13000 rpm (Heraeus Biofuge fresco, Festwinkelrotor 24) zentrifugiert. Der Überstand wurde bei 420nm gegen den Leerwert vermessen (Beckman DU 640 Spectrophotometer) und die β-Galactosidase-Aktivität berechnet:

$$U=1000\ A_{420}\ (CVt)^{-1}$$

$A_{420}$ ist dabei die Absorption bei 420 nm, C ist die Dichte der Zellsuspension ($OD_{600}$), V ist das eingesetzte Volumen der Zellsuspension und t die Inkubationszeit.

Die Messung wurde mit zwei weiteren Klonen wiederholt und der Mittelwert bestimmt.

| | | |
|---|---|---|
| Benötigte Lösungen: | Z-Puffer | 1000 ml:<br>16,1 g $Na_2HPO_4x7H_2O$<br>5,5 g $NaH_2PO_4x7H_2O$<br>0,75 g KCl<br>$MgSO_4x7H_2O$ |
| | Z-Puffer + Mercaptoethanol (frisch angesetzt) | 100 ml Z-Puffer + 270 µl Mercaptoethanol |

Beispiel 7: Rekonstitutionsassay Mg-Chelatase-Aktivität in Hefe (Willows et al., 1996, Eur.J.Biochem. 235,438-443) Stamm SFY526+pAS2-chlD+pACT2-chlH+pSG28

Es wurden kompetente Zellen des Stammes SFY526+pAS2-chlD+pACT2-chlH hergestellt (Klebe et al.; 1983; Gene, 25, 333-341) und mit pSG28 (p423TEF+chlI; p423TEF: Mumberg et al.; 1995; Gene, 156; 119-122) transformiert. Transformanten wurden auf Minimalagar (10 % YNB-Glukose; 1,5% Agar) unter Zusatz von Adenin (20mg/l) und L-Lysin-HCl (30mg/l) selektiert.
15 ml einer Vorkultur (Minimalmedium: 10% YNB-Glukose + 20 mg/l Adenin + 30 mg/l L-Lysin-HCl) dieses Stammes wurden 24 Stunden bei 30°C und 180 rpm in einer sterilen 50ml-Steilbrustflasche mit Metallkappe kultiviert.
150 ml Hauptkultur (Minimalmedium: 10% YNB-Glukose + 20 mg/l Adenin + 30 mg/l L-lysin-HCl) wurden mit 7,5 ml Vorkultur beimpft und 20 Stunden bei 30°C und 180 rpm unter Nutzung eines sterilen 500 ml-Erlenmeyerkolbens mit Wattestopfen geschüttelt. Die Zellen wurden 5 min bei 5000 rpm (Sigma 4K 10) sedimentiert und in 1,5 ml Assay-Puffer (0,1 M Tricin pH 7,9, 0,3 M Glycerol, 25 mM $MgCl_2$, 4 mM DTT) resuspendiert. Anschließend wurde diese Zellsuspension 3x30 Sekunden durch Ultraschall auf Eis bei geringer Ultraschallstärke aufgeschlossen.
Die aufgeschlossenen Zellen wurden 15 min bei 5000 rpm (Sigma 4 K 10) sedimentiert und es wurde mit dem Überstand weitergearbeitet. Der Proteingehalt dieses Hefeextraktes wurde unter Nutzung des BioRad Protein Assay nach der Methode von Bradford bestimmt.
Das Assayvolumen betrug 1 ml. 1 ml Assay-Puffer enthielten 1 mg Proteinextrakt der aufgeschlossenen Hefekultur, 4 mM ATP, 1,5 µM Protoporphyrin IX, 50 mM Phosphocreatin und 10 U Creatinphosphokinase.
Dieser Inkubationsmix wurde eine Stunde bei 30°C im Dunkeln inkubiert und danach ein Fluoreszenz-Emissions-Spektrum zwischen 500 und 650 nm bei einer anregenden Wellenlänge von 420 nm an einem Perkin Elmer Luminescence Spectrometer LS50B aufgenommen und einer HPLC-Analyse unterzogen.

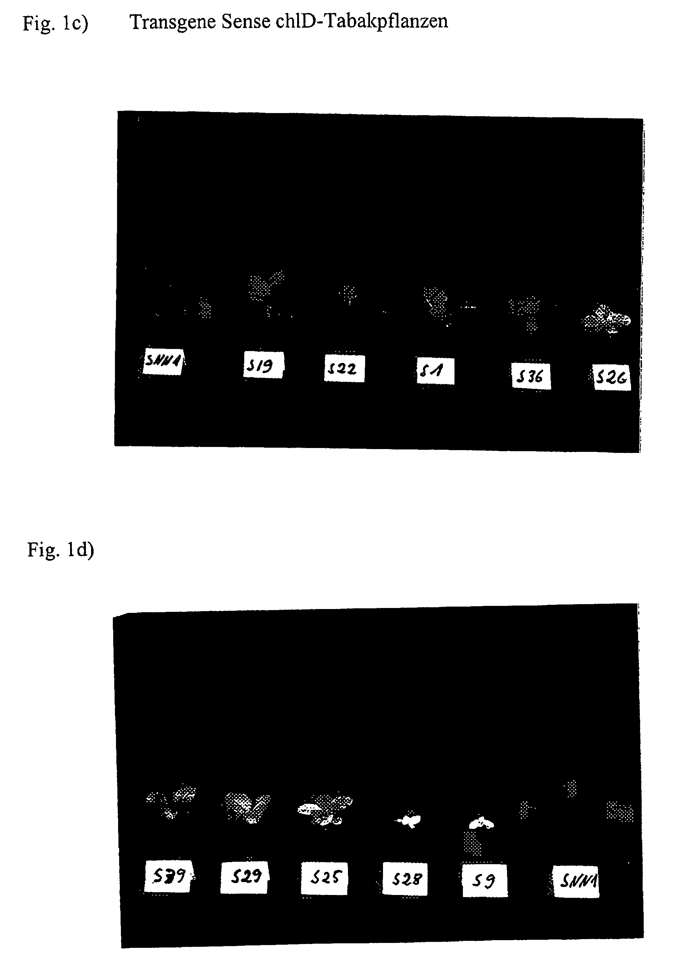

Fig. 1 (a - d) zeigt transgene chlD Sense- und Antisense-Tabakpflanzen

## Patentansprüche

1. Nukleinsäuremolekül, welches für eine CHLD-Untereinheit der pflanzlichen Mg-Chelatase kodiert, **dadurch gekennzeichnet, daß** seine Sequenz die Nukleinsäuresequenz gemäß SEQ ID NO:1 umfaßt.

2. Nukleinsäuremolekül gemäß Anspruch 1, **dadurch gekennzeichnet, daß** es für ein Protein gemäß SEQ ID NO: 2 kodiert.

3. Nukleinsäuremolekül, **dadurch gekennzeichnet, daß** es eine antisense oder komplementäre Sequenz des Nukleinsäuremoleküls mit der SEQ ID NO:1 darstellt.

4. Verwendung eines Nukleinsäuremoleküls gemäß einem oder mehreren der Ansprüche 1 bis 2 zur Herstellung von Antikörpern.

5. Verwendung eines Nukleinsäuremoleküls gemäß einem oder mehreren der Ansprüche 1 bis 3 zur Herstellung von transgenen Pflanzen.

6. CHLD-Untereinheit der pflanzlichen Mg-Chelatase, die durch ein Nukleinsäuremolekül gemäß einem der Ansprüche 1 oder 2 kodiert wird.

7. Protein gemäß Anspruch 6, **gekennzeichnet durch** SEQ ID Nr. 2.

8. Verwendung eines Proteins gemäß einem oder mehreren der Ansprüche 6 und 7 zur Aktivitätsbestimmung einer pflanzlichen Mg-Chelatase.

9. Verwendung eines Proteins gemäß einem oder mehreren der Ansprüche 6 und 7 zur Herstellung von Antikörpern.

10. Verfahren zur Herstellung der CHLD-Untereinheit einer pflanzlichen Mg-Chelatase, **dadurch gekennzeichnet, daß**:

    (a) eine Wirtszelle mit einem Nukleinsäuremolekül gemäß einem der Ansprüche 1 bis 2 transformiert wird

    (b) die gemäß (a) transformierte Wirtszelle in einem geeigneten Kulturmedium kultiviert wird, und

(c) die von der Wirtszelle gemäß (b) produzierte CHLD-Untereinheit der pflanzlichen Mg-Chelatase in geeigneter Weise aus dem Kulturmedium oder der Wirtszelle isoliert wird.

**11.** Verfahren zur Herstellung einer pflanzlichen Mg-Chelatase, **dadurch gekennzeichnet, daß**:

(a) eine Wirtszelle mit einem Nukleinsäuremolekül, gemäß einem der Ansprüche 1 bis 2 transformiert wird,

(b) die gemäß (a) transformierte Wirtszelle in einem geeigneten Kulturmedium kultiviert wird, und

(c) die von der Wirtszelle gemäß (b) produzierte CHLD-Untereinheit der pflanzlichen Mg-Chelatase in geeigneter Weise aus dem Kulturmedium oder der Wirtszelle isoliert wird.

**12.** Verfahren zur Bestimmung der Wechselwirkung pflanzlicher Mg-Chelatase Untereinheiten, **dadurch gekennzeichnet, daß**

(a) eine Wirtszelle mit einem Nukleinsäuremolekül gemäß einem oder mehreren der Ansprüche 1 bis 2 sowie mindestens mit einem Nukleinsäuremolekül kodierend für eine weitere Untereinheit der pflanzlichen Mg-Chelatase transformiert wird.

(b) die gemäß (a) transformierte Wirtszelle in einem geeigneten Kulturmedium kultiviert wird, und

(c) die Interaktion der pflanzlichen Mg-Chelatase zu einem direkt oder indirekt, qualitativ oder quantitativ meßbaren Signal führt.

**13.** Verfahren zur Bestimmung der Aktivität pflanzlicher Mg-Chelatase, **dadurch gekennzeichnet, daß** ein Protein gemäß einem oder mehreren der Ansprüche 7 und 8 mit den Genprodukten der DNA-Sequenzen kodierend für die pflanzlichen Mg-Chelatase-Untereinheiten I und H in einer Weise zusammen gebracht wird, daß die enzymatische Aktivität der Mg-Chelatase-Genprodukte zu einem direkt oder indirekt, qualitativ oder quantitativ meßbaren Signal führt.

**14.** Verwendung eines Verfahrens gemäß einem oder mehreren der Ansprüche 12 und 13 zur Identifizierung von Inhibitoren oder Aktivatoren der pflanzlichen Mg-Chelatase.

**15.** Nicht-natürlich vorkommendes chimäres Gen, enthaltend einen Promoter, der funktionell mit einem Nukleinsäuremolekül gemäß einem oder mehreren der Ansprüche 1 bis 3 fusioniert ist.

**16.** Vektor, enthaltend ein Nukleinsäuremolekül gemäß einem oder mehreren der Ansprüche 1 bis 3.

**17.** Rekombinante Wirtszelle, **dadurch gekennzeichnet, daß** diese Wirtszelle ein Nukleinsäuremolekül gemäß einem oder mehreren der Ansprüche 1 bis 3 exprimiert.

**18.** Transgene Pflanze, transgene Pflanzenzelle, transgene Pflanzenteile, transgene Pflanzensamen, transgenes Vermehrungsgut, enthaltend ein Nukleinsäuremolekül gemäß einem oder mehreren der Ansprüche 1 bis 3.

**19.** Verfahren zur Herstellung transgener Pflanzen, transgener Pflanzenzellen, transgener Pflanzenteile, transgener Pflanzensamen, transgenen Vermehrungsguts mittels einer rekombinanten Wirtszelle gemäß Anspruch 16.

## Claims

**1.** A nucleic acid molecule which encodes a plant Mg chelatase CHLD subunit and comprises the nucleic acid sequence in accordance with SEQ ID No. 1.

**2.** The nucleic acid molecule as claimed in claim 1 which encodes a protein in accordance with SEQ ID No. 2.

**3.** A nucleic acid molecule which represents an antisense or complementary sequence of the nucleic acid molecule with SEQ ID No. 1.

**4.** The use of a nucleic acid molecule as claimed in one or more of claims 1 to 2 for raising antibodies.

**5.** The use of a nucleic acid molecule as claimed in one or more of claims 1 to 3 for generating transgenic plants.

**6.** A plant Mg chelatase CHLD subunit which is encoded by a nucleic acid molecule as claimed in claim 1 or 2.

**7.** A protein as claimed in claim 6, in accordance with SEQ ID No. 2.

**8.** The use of the protein as claimed in one or more of claims 6 and 7 for determining the activity of a plant Mg chelatase.

**9.** The use of the protein as claimed in one or more of claims 6 and 7 for raising antibodies.

**10.** A method of generating the CHLD subunit of a plant Mg chelatase, which comprises:

(a) transforming a host cell with a nucleic acid molecule as claimed in one of claims 1 to 2,

(b) growing the host cell transformed in accordance with (a) in a suitable culture medium, and

(c) isolating the plant Mg chelatase CHLD subunit produced by the host cell in accordance with (b) in a suitable manner from the culture medium or the host cell.

**11.** A method of generating a plant Mg chelatase, which comprises:

(a) transforming a host cell with a nucleic acid molecule as claimed in one of claims 1 to 2,

(b) growing the host cell transformed in accordance with (a) in a suitable culture medium, and

(c) isolating the plant Mg chelatase CHLD subunit produced by the host cell in accordance with (b) in a suitable manner from the culture medium or the host cell.

**12.** A method for determining the interaction of plant Mg chelatase subunits, which comprises:

(a) transforming a host cell with a nucleic acid molecule as claimed in one or more of claims 1 to 2 and at least with one nucleic acid molecule encoding a further plant Mg chelatase subunit,

(b) growing the host cell transformed in accordance with (a) in a suitable culture medium, and

(c) the interaction of the plant Mg chelatase leading to a signal which can be measured directly or indirectly, qualitatively or quantitatively.

**13.** A method of determining the plant Mg chelatase activity which comprises combining a protein as claimed in one or more of claims 7 and 8 with the gene products of the DNA sequences encoding the plant Mg chelatase subunits I and H in such a way that the enzymatic activity of the Mg chelatase gene products leads to a directly or indirectly, qualitatively or quantitatively measurable signal.

**14.** The use of a method as claimed in one or more of claims 12 and 13 for identifying inhibitors or activators of plant Mg chelatase.

**15.** A non-naturally occurring chimeric gene comprising a promoter which is functionally fused to a nucleic acid molecule as claimed in one or more of claims 1 to 3.

**16.** A vector comprising a nucleic acid molecule as claimed in one or more of claims 1 to 3.

**17.** A recombinant host cell which expresses a nucleic acid molecule as claimed in one or more of claims 1 to 3.

**18.** A transgenic plant, transgenic plant cells, transgenic plant organs, transgenic plant seeds, transgenic propagation material comprising a nucleic acid molecule as claimed in one or more of claims 1 to 3.

19. A method of generating transgenic plants, transgenic plant cells, transgenic plant organs, transgenic plant seeds, transgenic propagation material by means of a recombinant host cell as claimed in claim 16.

**Revendications**

1. Molécule d'acide nucléique, qui code pour une sous-unité CHLD de la Mg-chélatase végétale, **caractérisée en ce que** sa séquence comprend la séquence en acides nucléiques selon la SEQ ID NO:1.

2. Molécule d'acide nucléique selon la revendication 1, **caractérisée en ce qu'**elle code pour une protéine selon la SEQ ID NO:2.

3. Molécule d'acide nucléique, **caractérisée en ce qu'**elle comprend une séquence antisens ou complémentaire de la molécule d'acide nucléique avec la SEQ ID NO:1.

4. Utilisation d'une molécule d'acide nucléique selon une ou plusieurs des revendications 1 à 2, pour la préparation d'anticorps.

5. Utilisation d'une molécule d'acide nucléique selon une ou plusieurs des revendications 1 à 3, pour la préparation de plantes transgéniques.

6. Sous-unité CHLD de la Mg-chélatase végétale, qui est codée par une molécule d'acide nucléique selon une ou plusieurs des revendications 1 ou 2.

7. Protéine selon la revendication 6, **caractérisée par** la SEQ ID NO:2.

8. Utilisation d'une protéine selon une ou plusieurs des revendications 6 et 7 pour la détermination de l'activité d'une Mg-chélatase végétale.

9. Utilisation d'une protéine selon une ou plusieurs des revendications 6 et 7 pour la préparation d'anticorps.

10. Procédé pour la préparation de la sous-unité CHLD d'une Mg-chélatase végétale, **caractérisé en ce que** :

    (a) une cellule hôte est transformée par une molécule d'acide nucléique selon l'une des revendications 1 à 2,
    (b) la cellule hôte transformée selon (a) est mise en culture dans un milieu de culture approprié, et
    (c) la sous-unité CHLD de la Mg-chélatase végétale produite par la cellule hôte selon (b) est isolée de façon appropriée à partir du milieu de culture ou de la cellule hôte.

11. Procédé pour la préparation d'une Mg-chélatase végétale, **caractérisé en ce que** :

    (a) une cellule hôte est transformée par une molécule d'acide nucléique selon l'une des revendications 1 à 2,
    (b) la cellule hôte transformée selon (a) est mise en culture dans un milieu de culture approprié, et
    (c) la sous-unité CHLD de la Mg-chélatase végétale produite par la cellule hôte selon (b) est isolée de façon appropriée à partir du milieu de culture ou de la cellule hôte.

12. Procédé pour la détermination de l'interaction des sous-unités de la Mg-chélatase végétale, **caractérisé en ce que** :

    (a) une cellule hôte est transformée par une molécule d'acide nucléique selon une ou plusieurs des revendications 1 à 2 ainsi qu'au moins par une molécule d'acide nucléique codante pour une autre sous-unité de la Mg-chélatase végétale,
    (b) la cellule hôte transformée selon (a) est mise en culture dans un milieu de culture approprié, et
    (c) l'interaction de la Mg-chélatase végétale conduit à un signal direct ou indirect, mesurable qualitativement ou quantitativement.

13. Procédé pour la détermination de l'activité de la Mg-chélatase végétale, **caractérisé en ce qu'**on réunit une protéine selon une ou plusieurs des revendications 7 et 8 avec les produits de gène des séquences d'ADN codantes pour les sous-unités I et H de la Mg-chélatase végétale d'une façon telle que l'activité enzymatique des produits de gène de la Mg-chélatase conduit à un signal direct ou indirect, mesurable qualitativement ou quantitativement.

**14.** Utilisation d'un procédé selon une ou plusieurs des revendications 12 et 13 pour l'identification d'inhibiteurs ou d'activateurs de la Mg-chélatase végétale.

**15.** Gène chimère ne se trouvant pas dans la nature contenant un promoteur qui est fonctionnellement fusionné avec une molécule d'acide nucléique selon une ou plusieurs des revendications 1 à 3.

**16.** Vecteur renfermant une molécule d'acide nucléique selon une ou plusieurs des revendications 1 à 3.

**17.** Cellule hôte recombinante, **caractérisée en ce que** cette cellule hôte exprime une molécule d'acide nucléique selon une ou plusieurs des revendications 1 à 3.

**18.** Plante transgénique, cellule de plante transgénique, semence de plante transgénique, produit de multiplication transgénique, renfermant une molécule d'acide nucléique selon une ou plusieurs des revendications 1 à 3.

**19.** Procédé pour la préparation de plantes transgéniques, de cellules de plantes transgéniques, de semences de plantes transgéniques, de produit de multiplication transgénique, à l'aide d'une cellule hôte recombinante selon la revendication 16.

Fig. 1a) Transgene Antisense chlD-Tabakpflanzen

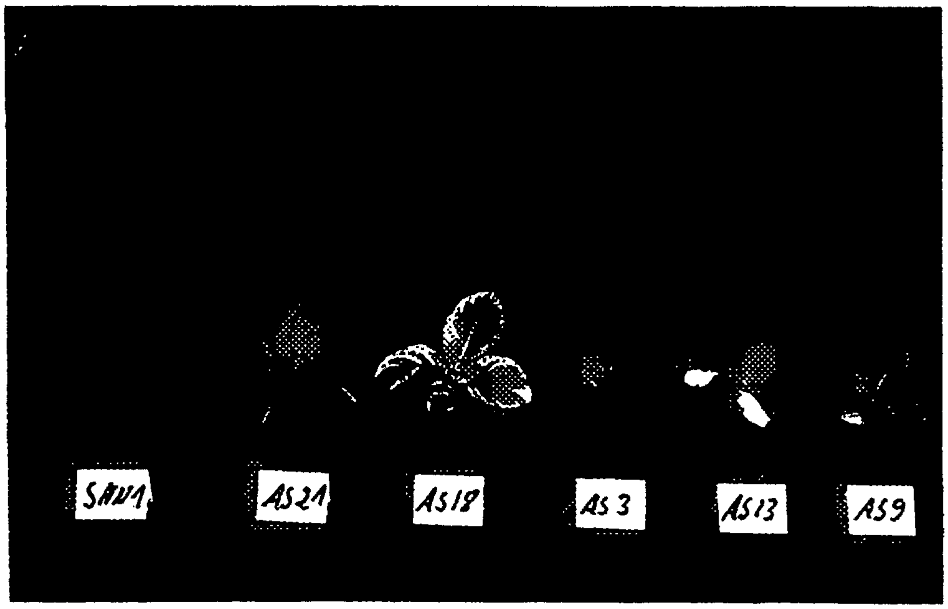


Fig. 1b)

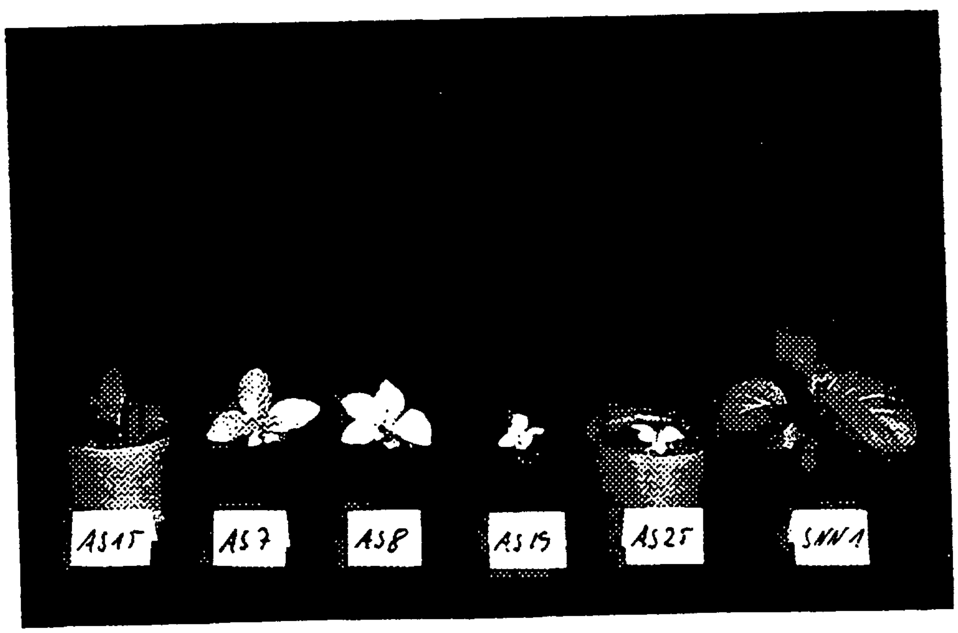

Fig. 1c) Transgene Sense chlD-Tabakpflanzen

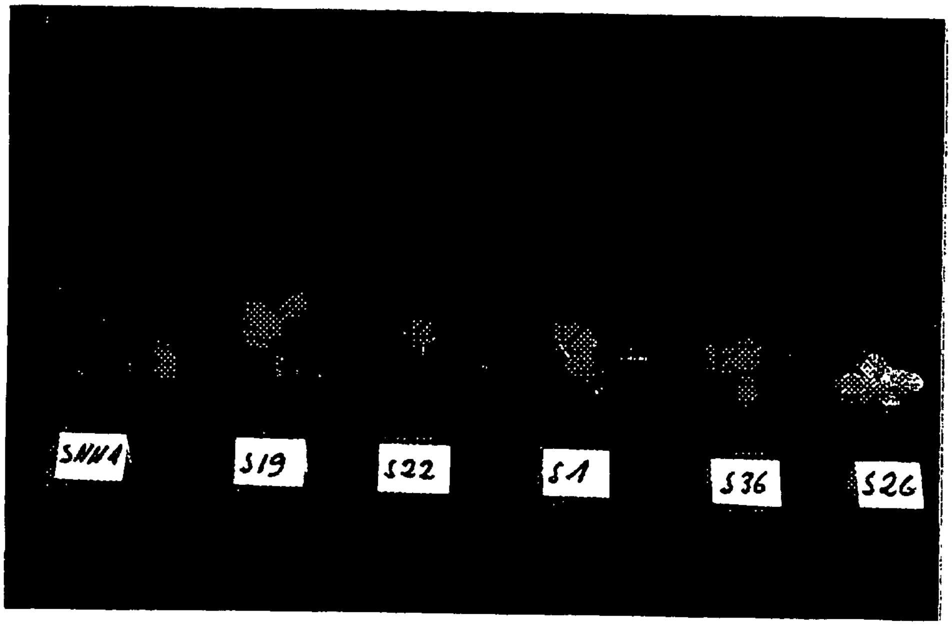


Fig. 1d)

SEQUENZPROTOKOLL

(1) ALLGEMEINE INFORMATION:

(i) ANMELDER:

(A) NAME: Hoechst Schering AgrEvo GmbH
(B) STRASSE: -
(C) ORT: Frankfurt
(D) BUNDESLAND: -
(E) LAND: Deutschland
(F) POSTLEITZAHL: 65926
(G) TELEPHON: 069-305-82808

(H) TELEFAX: 069-35-7175
(I) TELEX: -

(ii) ANMELDETITEL: DNA-Sequenzen kodierend fuer die Untereinheit CHLD pflanzlicher Magnesium-Chelatasen sowie Verfahren zur Aktivitaetsbestimmung pflanzlicher Magnesium-Chelatasen

(iii) ANZAHL DER SEQUENZEN: 7

(iv) COMPUTER-LESBARE FORM:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:

(A) LÄNGE: 2495 Basenpaare
(B) ART: Nukleinsäure
(C) STRANGFORM: Einzel
(D) TOPOLOGIE: linear

(ii) ART DES MOLEKULS: DNS (genomisch)

(ix) MERKMALE:

(A) NAME/SCHLÜSSEL: exon
(B) LAGE: 1..39

(ix) MERKMALE:

(A) NAME/SCHLÜSSEL: CDS
(B) LAGE: 40..2313

(ix) MERKMALE:

(A) NAME/SCHLÜSSEL: exon
(B) LAGE: 41..2495

(xi) SEQUENZBESCHREIBUNG: SEQ ID NO: 1:

```
CATCTAAAAT CCTAAATCAA AAACTTCGAT GCTATAAAA ATG GGG TTT TGT TCA       54
                                           Met Gly Phe Cys Ser
                                             1               5

ACT TCA ACC CTC CCA CAA ACA TCA CTA TCC AAT TCT CAA TCT TCA ACA     102
Thr Ser Thr Leu Pro Gln Thr Ser Leu Ser Asn Ser Gln Ser Ser Thr
                 10                  15                  20

TTC TTC ACA TAC TTA AAA CCA TGC CCA ATT CTA TCC TCC ACA TAT TTA     150
```

```
Phe Phe Thr Tyr Leu Lys Pro Cys Pro Ile Leu Ser Ser Thr Tyr Leu
            25                  30                  35

AGG CCG GAA CGG CTA AAA TTT CGC CTC AGA ATA AGT GCC ACT GCA ACT        198
Arg Pro Glu Arg Leu Lys Phe Arg Leu Arg Ile Ser Ala Thr Ala Thr
        40                  45                  50

ATT GAT TCA CCT AAT GGC GCT GTT GCA GTA GTG GAA CCT GAA AAA CAA        246
Ile Asp Ser Pro Asn Gly Ala Val Ala Val Val Glu Pro Glu Lys Gln
    55                  60                  65

CCT GAG AAA ATT TCC TTT GGT AGA CAG TAT TTT CCT CTA GCT GCT GTT        294
Pro Glu Lys Ile Ser Phe Gly Arg Gln Tyr Phe Pro Leu Ala Ala Val
70                  75                  80                  85

ATT GGA CAG GAT GCT ATT AAA ACT GCT CTT TTA CTT GGG GCC ATT GAC        342
Ile Gly Gln Asp Ala Ile Lys Thr Ala Leu Leu Leu Gly Ala Ile Asp
                90                  95                  100

CGT GAG ATA GGA GGA ATT GCA ATA TGT GGG AAG CGT GGA ACA GCG AAA        390
Arg Glu Ile Gly Gly Ile Ala Ile Cys Gly Lys Arg Gly Thr Ala Lys
            105                 110                 115

ACG TTA ATG GCA CGT GGA TTG CAT GCT ATT CTG CCA CCA ATT GAA GTA        438
Thr Leu Met Ala Arg Gly Leu His Ala Ile Leu Pro Pro Ile Glu Val
        120                 125                 130

GTT GTT GGC TCA ATG GCA AAT GCT GAT CCG AAC TGT CCC GAT GAG TGG        486
Val Val Gly Ser Met Ala Asn Ala Asp Pro Asn Cys Pro Asp Glu Trp
    135                 140                 145

GAA GAC GGG CTA GCT GAC AGA GCA GAA TAT GGG TCT GAT GGT AAT ATC        534
Glu Asp Gly Leu Ala Asp Arg Ala Glu Tyr Gly Ser Asp Gly Asn Ile
150                 155                 160                 165

AAG ACC CAG ATA GTT AAA TCC CCA TTT GTT CAG ATT CCC CTT GGT GTC        582
Lys Thr Gln Ile Val Lys Ser Pro Phe Val Gln Ile Pro Leu Gly Val
                170                 175                 180

ACA GAA GAT AGA TTG ATT GGC TCT GTT GAT GTC GAG GAG TCC GTG AAA        630
Thr Glu Asp Arg Leu Ile Gly Ser Val Asp Val Glu Glu Ser Val Lys
            185                 190                 195

TCT GGA ACC ACT GTC TTT CAA CCA GGC CTC CTC GCA GAA GCT CAT CGA        678
Ser Gly Thr Thr Val Phe Gln Pro Gly Leu Leu Ala Glu Ala His Arg
        200                 205                 210

GGA GTT CTA TAT GTT GAT GAG ATT AAT CTA TTA GAT GAA GGT ATA AGT        726
Gly Val Leu Tyr Val Asp Glu Ile Asn Leu Leu Asp Glu Gly Ile Ser
    215                 220                 225

AAC CTA CTT CTG AAT GTA TTG ACT GAG GGA GTC AAT ATT GTA GAA AGA        774
Asn Leu Leu Leu Asn Val Leu Thr Glu Gly Val Asn Ile Val Glu Arg
230                 235                 240                 245

GAG GGA ATC AGC TTT CGA CAT CCA TGC AAA CCA CTA CTA ATT GCT ACC        822
Glu Gly Ile Ser Phe Arg His Pro Cys Lys Pro Leu Leu Ile Ala Thr
                250                 255                 260

TAT AAC CCT GAA GAG GGT GCG GTT CGT GAG CAT CTG CTA GAC CGT ATT        870
Tyr Asn Pro Glu Glu Gly Ala Val Arg Glu His Leu Leu Asp Arg Ile
            265                 270                 275
```

```
GCG ATT AAT TTA AGT GCA GAT CTT CCA ATG AGT TTT GAC GAT CGT GTT      918
Ala Ile Asn Leu Ser Ala Asp Leu Pro Met Ser Phe Asp Asp Arg Val
        280                 285                 290

GCA GCT GTT GAC ATA GCA ACA CGT TTT CAG GAG TGT AGC AAT GAG GTT      966
Ala Ala Val Asp Ile Ala Thr Arg Phe Gln Glu Cys Ser Asn Glu Val
    295                 300                 305

TTT AAA ATG GTG GAT GAA GAA ACA GAC AGT GCA AAA ACC CAG ATA ATA     1014
Phe Lys Met Val Asp Glu Glu Thr Asp Ser Ala Lys Thr Gln Ile Ile
310                 315                 320                 325

TTG GCA AGG GAG TAT TTA AAG GAT GTC ACA ATC AGT AGA GAT CAA CTA     1062
Leu Ala Arg Glu Tyr Leu Lys Asp Val Thr Ile Ser Arg Asp Gln Leu
                330                 335                 340

AAA TAC TTG GTC ATG GAA GCA ATT CGT GGT GGC TGC CAG GGG CAC CGA     1110
Lys Tyr Leu Val Met Glu Ala Ile Arg Gly Gly Cys Gln Gly His Arg
            345                 350                 355

GCT GAA CTT TAT GCT GCT CGT GTA GCC AAA TGC TTA GCT GCC ATC GAT     1158
Ala Glu Leu Tyr Ala Ala Arg Val Ala Lys Cys Leu Ala Ala Ile Asp
        360                 365                 370

GGA CGT GAA AAA GTT GGT GTT GAT GAG CTG AAA AAA GCT GTA GAG CTT     1206
Gly Arg Glu Lys Val Gly Val Asp Glu Leu Lys Lys Ala Val Glu Leu
    375                 380                 385

GTC ATC CTC CCA CGT TCA ACT ATA GTT GAA AAC CCA CCA GAC CAG CAA     1254
Val Ile Leu Pro Arg Ser Thr Ile Val Glu Asn Pro Pro Asp Gln Gln
390                 395                 400                 405

AAC CAG CAG CCA CCT CCT CCC CCT CCC CCT CCC CAA AAT CAA GAT TCT     1302
Asn Gln Gln Pro Pro Pro Pro Pro Pro Pro Pro Gln Asn Gln Asp Ser
                410                 415                 420

TCA GAA GAG CAG AAT GAA GAA GAA GAA AAA GAA GAA GAA GAT CAA GAG     1350
Ser Glu Glu Gln Asn Glu Glu Glu Glu Lys Glu Glu Glu Asp Gln Glu
            425                 430                 435

GAT GAG AAA GAT AGA GAA AAT GAA CAG CAA CAG CCA CAA GTC CCT GAT     1398
Asp Glu Lys Asp Arg Glu Asn Glu Gln Gln Gln Pro Gln Val Pro Asp
        440                 445                 450

GAG TTT ATT TTT GAT GCG GAA GGT GGT TTA GTG GAT GAA AAA CTT CTC     1446
Glu Phe Ile Phe Asp Ala Glu Gly Gly Leu Val Asp Glu Lys Leu Leu
    455                 460                 465

TTC TTT GCA CAA CAA GCA CAA AGA CGC AAA GGA AAA GCT GGA CGA GCA     1494
Phe Phe Ala Gln Gln Ala Gln Arg Arg Lys Gly Lys Ala Gly Arg Ala
470                 475                 480                 485

AAG AAG GTC ATC TTT TCC GAA GAT AGA GGT CGA TAT ATA AAG CCA ATG     1542
Lys Lys Val Ile Phe Ser Glu Asp Arg Gly Arg Tyr Ile Lys Pro Met
                490                 495                 500

CTT CCA AAG GGT CCA GTG AAG AGA TTG GCA GTT GAT GCA ACT CTA AGA     1590
Leu Pro Lys Gly Pro Val Lys Arg Leu Ala Val Asp Ala Thr Leu Arg
            505                 510                 515

GCA GCG GCA CCA TAT CAG AAG TTA CGA AGA GCA AAG GAC ATC CAA AAA     1638
Ala Ala Ala Pro Tyr Gln Lys Leu Arg Arg Ala Lys Asp Ile Gln Lys
        520                 525                 530
```

```
ACT CGC AAG GTT TAT GTA GAG AAA ACT GAC ATG AGA GCC AAA AGA ATG      1686
Thr Arg Lys Val Tyr Val Glu Lys Thr Asp Met Arg Ala Lys Arg Met
    535                 540                 545

GCA CGC AAA GCC GGA GCT CTG GTG ATA TTC GTA GTT GAC GCT AGT GGG      1734
Ala Arg Lys Ala Gly Ala Leu Val Ile Phe Val Val Asp Ala Ser Gly
550                 555                 560                 565

AGT ATG GCA CTG AAT AGA ATG CAG AAT GCC AAA GGA GCA GCA CTT AAA      1782
Ser Met Ala Leu Asn Arg Met Gln Asn Ala Lys Gly Ala Ala Leu Lys
                570                 575                 580

CTA CTT GCA GAG AGT TAT ACA AGC AGA GAT CAG GTC TGT ATC ATT CCC      1830
Leu Leu Ala Glu Ser Tyr Thr Ser Arg Asp Gln Val Cys Ile Ile Pro
            585                 590                 595

TTC CGC GGA GAT GCT GCT GAA GTT TTG TTG CCA CCT TCT AGG TCA ATA      1878
Phe Arg Gly Asp Ala Ala Glu Val Leu Leu Pro Pro Ser Arg Ser Ile
        600                 605                 610

TCG ATG GCA AGA AAT CGT CTT GAG AGA CTT CCC TGT GGA GGG GGT TCT      1926
Ser Met Ala Arg Asn Arg Leu Glu Arg Leu Pro Cys Gly Gly Gly Ser
    615                 620                 625

CCC CTT GCT CAT GGG CTT ACG ACG GCA GTT AGA GTT GGA ATG AAT GCA      1974
Pro Leu Ala His Gly Leu Thr Thr Ala Val Arg Val Gly Met Asn Ala
630                 635                 640                 645

GAA AAG AGT GGT GAT GTT GGA CGT ATC ATG ATT GTT GCA ATT ACT GAT      2022
Glu Lys Ser Gly Asp Val Gly Arg Ile Met Ile Val Ala Ile Thr Asp
                650                 655                 660

GGT AGA GCT AAC ATC TCT CTT AAA AGA TCC ACA GAC CCT GAA GCT GAA      2070
Gly Arg Ala Asn Ile Ser Leu Lys Arg Ser Thr Asp Pro Glu Ala Glu
            665                 670                 675

GCT TCT GAT GCA CCC AGA CCT TCT TCC CAA GAG CTG AAG GAT GAG ATT      2118
Ala Ser Asp Ala Pro Arg Pro Ser Ser Gln Glu Leu Lys Asp Glu Ile
        680                 685                 690

CTC GAG GTG GCT GGT AAA ATA TAC AAA ACA GGA ATG TCT CTC CTC GTC      2166
Leu Glu Val Ala Gly Lys Ile Tyr Lys Thr Gly Met Ser Leu Leu Val
    695                 700                 705

ATA GAT ACA GAA AAT AAG TTT GTT TCT ACT GGT TTT GCG AAA GAA ATC      2214
Ile Asp Thr Glu Asn Lys Phe Val Ser Thr Gly Phe Ala Lys Glu Ile
710                 715                 720                 725

GCG AGA GTA GCT CAA GGG AAG TAC TAT TAT TTA CCA AAT GCT TCA GAT      2262
Ala Arg Val Ala Gln Gly Lys Tyr Tyr Tyr Leu Pro Asn Ala Ser Asp
                730                 735                 740

GCT GTG ATA TCT GCA GCA ACA AAG GAT GCA TTA TCT GCA TTA AAG GAA      2310
Ala Val Ile Ser Ala Ala Thr Lys Asp Ala Leu Ser Ala Leu Lys Glu
            745                 750                 755

TCT TGACCTAAAC TCGATCGAAT TAATTGTAAA TGTTGTTTTG AGTATAGATT           2363
Ser

ATTGGGAGGA TATAAGAGCT TGCTTGATAA TTCTTATCTT TTGTTGTACT AATTGAACTT    2423
```

ATTTCTCAAT TATGCAATCA GGGTAATGAA GATTCTTTTC ATTTCAAAAA AAAAAAAAAA 2483

AAAGGAATTC GA 2495

(2) INFORMATION ZU SEQ ID NO: 2:

(i) SEQUENZ CHARAKTERISTIKA:

(A) LÄNGE: 758 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 2:

Met Gly Phe Cys Ser Thr Ser Thr Leu Pro Gln Thr Ser Leu Ser Asn
1 5 10 15

Ser Gln Ser Ser Thr Phe Phe Thr Tyr Leu Lys Pro Cys Pro Ile Leu
20 25 30

Ser Ser Thr Tyr Leu Arg Pro Glu Arg Leu Lys Phe Arg Leu Arg Ile
35 40 45

Ser Ala Thr Ala Thr Ile Asp Ser Pro Asn Gly Ala Val Ala Val Val
50 55 60

Glu Pro Glu Lys Gln Pro Glu Lys Ile Ser Phe Gly Arg Gln Tyr Phe
65 70 75 80

Pro Leu Ala Ala Val Ile Gly Gln Asp Ala Ile Lys Thr Ala Leu Leu
85 90 95

Leu Gly Ala Ile Asp Arg Glu Ile Gly Gly Ile Ala Ile Cys Gly Lys
100 105 110

Arg Gly Thr Ala Lys Thr Leu Met Ala Arg Gly Leu His Ala Ile Leu
115 120 125

Pro Pro Ile Glu Val Val Val Gly Ser Met Ala Asn Ala Asp Pro Asn
130 135 140

Cys Pro Asp Glu Trp Glu Asp Gly Leu Ala Asp Arg Ala Glu Tyr Gly
145 150 155 160

Ser Asp Gly Asn Ile Lys Thr Gln Ile Val Lys Ser Pro Phe Val Gln
165 170 175

Ile Pro Leu Gly Val Thr Glu Asp Arg Leu Ile Gly Ser Val Asp Val
180 185 190

Glu Glu Ser Val Lys Ser Gly Thr Thr Val Phe Gln Pro Gly Leu Leu
195 200 205

Ala Glu Ala His Arg Gly Val Leu Tyr Val Asp Glu Ile Asn Leu Leu
210 215 220

Asp Glu Gly Ile Ser Asn Leu Leu Leu Asn Val Leu Thr Glu Gly Val
225 230 235 240

Asn Ile Val Glu Arg Glu Gly Ile Ser Phe Arg His Pro Cys Lys Pro
245 250 255

```
Leu Leu Ile Ala Thr Tyr Asn Pro Glu Glu Gly Ala Val Arg Glu His
            260                 265                 270

Leu Leu Asp Arg Ile Ala Ile Asn Leu Ser Ala Asp Leu Pro Met Ser
        275                 280                 285

Phe Asp Asp Arg Val Ala Ala Val Asp Ile Ala Thr Arg Phe Gln Glu
    290                 295                 300

Cys Ser Asn Glu Val Phe Lys Met Val Asp Glu Glu Thr Asp Ser Ala
305                 310                 315                 320

Lys Thr Gln Ile Ile Leu Ala Arg Glu Tyr Leu Lys Asp Val Thr Ile
                325                 330                 335

Ser Arg Asp Gln Leu Lys Tyr Leu Val Met Glu Ala Ile Arg Gly Gly
            340                 345                 350

Cys Gln Gly His Arg Ala Glu Leu Tyr Ala Ala Arg Val Ala Lys Cys
        355                 360                 365

Leu Ala Ala Ile Asp Gly Arg Glu Lys Val Gly Val Asp Glu Leu Lys
    370                 375                 380

Lys Ala Val Glu Leu Val Ile Leu Pro Arg Ser Thr Ile Val Glu Asn
385                 390                 395                 400

Pro Pro Asp Gln Gln Asn Gln Gln Pro Pro Pro Pro Pro Pro Pro Pro
                405                 410                 415

Gln Asn Gln Asp Ser Ser Glu Glu Gln Asn Glu Glu Glu Glu Lys Glu
            420                 425                 430

Glu Glu Asp Gln Glu Asp Glu Lys Asp Arg Glu Asn Glu Gln Gln Gln
        435                 440                 445

Pro Gln Val Pro Asp Glu Phe Ile Phe Asp Ala Glu Gly Gly Leu Val
    450                 455                 460

Asp Glu Lys Leu Leu Phe Phe Ala Gln Gln Ala Gln Arg Arg Lys Gly
465                 470                 475                 480

Lys Ala Gly Arg Ala Lys Lys Val Ile Phe Ser Glu Asp Arg Gly Arg
                485                 490                 495

Tyr Ile Lys Pro Met Leu Pro Lys Gly Pro Val Lys Arg Leu Ala Val
            500                 505                 510

Asp Ala Thr Leu Arg Ala Ala Ala Pro Tyr Gln Lys Leu Arg Arg Ala
        515                 520                 525

Lys Asp Ile Gln Lys Thr Arg Lys Val Tyr Val Glu Lys Thr Asp Met
    530                 535                 540

Arg Ala Lys Arg Met Ala Arg Lys Ala Gly Ala Leu Val Ile Phe Val
545                 550                 555                 560

Val Asp Ala Ser Gly Ser Met Ala Leu Asn Arg Met Gln Asn Ala Lys
                565                 570                 575

Gly Ala Ala Leu Lys Leu Leu Ala Glu Ser Tyr Thr Ser Arg Asp Gln
            580                 585                 590
```

```
Val Cys Ile Ile Pro Phe Arg Gly Asp Ala Ala Glu Val Leu Leu Pro
        595                 600                 605

Pro Ser Arg Ser Ile Ser Met Ala Arg Asn Arg Leu Glu Arg Leu Pro
    610                 615                 620

Cys Gly Gly Gly Ser Pro Leu Ala His Gly Leu Thr Thr Ala Val Arg
625                 630                 635                 640

Val Gly Met Asn Ala Glu Lys Ser Gly Asp Val Gly Arg Ile Met Ile
                645                 650                 655

Val Ala Ile Thr Asp Gly Arg Ala Asn Ile Ser Leu Lys Arg Ser Thr
            660                 665                 670

Asp Pro Glu Ala Glu Ala Ser Asp Ala Pro Arg Pro Ser Ser Gln Glu
        675                 680                 685

Leu Lys Asp Glu Ile Leu Glu Val Ala Gly Lys Ile Tyr Lys Thr Gly
    690                 695                 700

Met Ser Leu Leu Val Ile Asp Thr Glu Asn Lys Phe Val Ser Thr Gly
705                 710                 715                 720

Phe Ala Lys Glu Ile Ala Arg Val Ala Gln Gly Lys Tyr Tyr Tyr Leu
                725                 730                 735

Pro Asn Ala Ser Asp Ala Val Ile Ser Ala Ala Thr Lys Asp Ala Leu
            740                 745                 750

Ser Ala Leu Lys Glu Ser
        755
```

(2) INFORMATION ZU SEQ ID NO: 3:

(i) SEQUENZ CHARAKTERISTIKA:

(A) LÄNGE: 6 Aminosäuren
(B) ART: Aminosäure
(C) STRANGFORM: Einzel
(D) TOPOLOGIE: linear

(ii) ART DES MOLEKÜLS: Protein

(ix) MERKMALE:

(A) NAME/SCHLÜSSEL: Protein
(B) LAGE: 1..6

(xi) SEQUENZBESCHREIBUNG: SEQ ID NO: 3:

```
Val Asp Ala Ser Gly Ser
1               5
```

(2) INFORMATION ZU SEQ ID NO: 4:

(i) SEQUENZ CHARAKTERISTIKA:

(A) LÄNGE: 6 Aminosäuren

(B) ART: Aminosäure
(C) STRANGFORM: Einzel
(D) TOPOLOGIE: linear

(ii) ART DES MOLEKÜLS: Protein

(ix) MERKMALE:

(A) NAME/SCHLÜSSEL: Protein
(B) LAGE: 1..6

(xi) SEQUENZBESCHREIBUNG: SEQ ID NO: 4:

```
Thr Asp Gly Arg Gly Asn
1               5
```

(2) INFORMATION ZU SEQ ID NO: 5:

(i) SEQUENZ CHARAKTERISTIKA:

(A) LÄNGE: 1579 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:

(A) NAME/SCHLÜSSEL: exon
(B) LAGE: 1..1579

(xi) SEQUENZBESCHREIBUNG: SEQ ID NO: 5:

```
CCCAAAATTC TTCTTCTTCT TCTTCACTGA AAAATTCTAA ACAAAATGGC TTCACTACTA   60
GGAACTTCCT CTTCAGCAGC AGCTGCAATA TTAGCTTCTA CACCCTTGTC TTCTCGCTCC  120
TGTAAGCCTG CCGTTTTCTC CCTCTTCCCT TCTTCAGGGC AGAGTCAAGG GAGGAAGTTT  180
TATGGAGGGA TTAGAGTCCC AGTTAAGAAA GGGAGGTCCC AATTCCATGT GGCAATTTCA  240
AATGTTGCGA CGGAAATCAA CCTGCTCAAG AACAGGGTCA GAAACTTGCT GGAGGAGAGC  300
CAGAGACCGG TGTATCCATT TGCAGCTATA GTGGGACAAG ATGAAATGAA GTTATGTCTT  360
TTGCTGAATG TAATTGATCC AAAGATTGGA GGTGTGATGA TAATGGGTGA TAGGGGAACC  420
GGGAAGTCCA CCACGGTTAG ATCTTTGGTA GATTTACTTC CTGAAATCAA AGTTATTTCT  480
GGTGATCCGT TCAATTCAGA TCCAGATGAC CAAGAAGTAA TGAGTGCAGA AGTCCGTGAC  540
AAATTGAGGA GCGGACAGCA GCTTCCTATA TCTCGTACGA AAATCAACAT GGTTGATTTA  600
CCGCTTGGTG CTACTGAGGA CAGGGTGTGT GGCACAATCG ACATTGAGAA AGCTCTTACT  660
GAGGGTGTGA AGGCTTTCGA GCCTGGTCTT CTTGCTAAAG CTAACAGAGG AATACTTTAC  720
GTCGATGAGG TTAATCTTTT GGACGACCAT TTAGTAGATG TTCTTTTGGA TTCTGCAGCA  780
TCGGGATGGA ACACTGTTGA AAGAGAGGGG ATATCAATAT CACACCCTGC CCGGTTTATC  840
CTTATTGGTT CGGGTAATCC TGAAGAAGGA GAACTTAGGC CACAACTTCT TGATCGATTT  900
GGAATGCATG CCCAAGTGGG GACCGTGAGA GATGCAGAGC TGAGAGTGAA GATAGTTGAG  960

GAAAGAGCTC GTTTTGATAA GAACCCCAAG GAATTCAGGG AATCATACAA GGCAGAGCAA  1020
GAAAAGCTCC AGAACCAAAT CGACTCAGCT AGGAACGCTC TTTCTGCTGT TACAATCGAT  1080
CATGATCTTC GAGTTAAAAT CTCTAAGGTC TGTGCAGAAC TAAACGTCGA TGGATTGAGA  1140
GGTGATATAG TCACTAACAG GGCAGCAAGA GCGTTGGCTG CACTAAAAGG AAGAGATAAG  1200
GTAACTCCGG AAGATATCGC CACTGTCATT CCCAACTGCT TAAGACACAG GCTGAGGAAG  1260
GATCCGTTGG AATCTATTGA CTCGGGTGTA CTTGTTGTTG AGAAATTTTA TGAGGTTTTC  1320
GCCTAAGCGT TTTATAGAGT GAGATACTTA TTTTTGGCTT TATTTTCCAT TCATAAATCA  1380
TCTAAAGATT TGACAATTGT AACACTAGAC TTTTGCTTAA TTTTGGCTTT GTACTGTGCT  1440
TAAGAAATGG GTTCAGAATT ACCTGTAGCC AGTTGTATTT GGTTATGACT GCTTTATTTC  1500
TGAAATGCTT AAAAAAAAAA AAAAAAAAAA AAAAAAAAAA AAAAAAAAAA AAAAAAAAAA  1560
AAAAAAAAAA AAGGAATTC                                              1579
```

(2) INFORMATION ZU SEQ ID NO: 6:

(i) SEQUENZ CHARAKTERISTIKA:

(A) LÄNGE: 4578 Basenpaare
(B) ART: Nukleinsäure
(C) STRANGFORM: Einzel

(D) TOPOLOGIE: linear

(ii) ART DES MOLEKÜLS: DNS (genomisch)

(ix) MERKMALE:

(A) NAME/SCHLÜSSEL: exon
(B) LAGE: 1..4578

(xi) SEQUENZBESCHREIBUNG: SEQ ID NO: 6:

```
AGCCACTACT CTCCACATAA AACTATAAAC TTAGAACATT TCACTTGAAA AAAGAGAGGA     60
AAAAAGTGAA GCAGAAATCT TTTCTCAAAA CACAATCTAT AGGAAGTTAA ATTCAACTTC    120
CACACTTCCA AGATTCTTGT TTCAAGTTTC GTTTAGTTTT TTTTTCTTGG TTTTTTTTAT    180
AGTTTTCTGT ACAATTTTGT GTAGAATCAA GAAACGAAAG AGTTAAAGTT TGAAACTTTT    240
TTACAAGTTT GAAACAATGG CTTCTTTGGT TTCTTCACCA TTTACATTGC CAAATTCAAA    300
AGTAGAACAC TTGTCATCCA TTTCTCAAAA GCATTACTTT CTTCACTCAT TTCTTCCCAA    360
GAAAATAAAC CCCACTTACT CAAAATCACC AAAGAAATTC CAATGTAATG CTATTGGCAA    420
TGGTTTATTC ACTCAAACAA CTCAAGAAGT TAGGAGAATT GTGCCTGAAA ATACTCAGGG    480
ACTTGCTACT GTGAAAATAG TCTATGTTGT ATTGGAAGCT CAGTACCAAT CATCACTTAC    540
TGCTGCTGTT CAGACACTGA ACAAGAATGG TCAGTTTGCT TCTTTTGAGG TTGTGGGGTA    600
CTTGGTTGAG GAGCTTAGAG ATGAGAATAC TTATAAAATG TTTTGTAAAG ATCTTGAGGA    660
TGCAAATGTG TTTATTGGTT CATTGATTTT TGTGGAAGAA TTGGCTTTAA AGGTAAAATC    720
```

TGCAGTGGAG AAAGAAAGGG ACAGACTTGA TGCAGTTTTG GTGTTTCCAT CAATGCCTGA 780
GGTGATGAGG TTGAACAAGT TGGGATCTTT TAGTATGTCA CAATTGGGGC AATCAAAGAG 840
TCCATTTTTT GAGCTTTTCA AGAAGAAGAA ACCTTCTTCT GCAGGTTTTT CTGATCAGAT 900
GTTGAAGCTT GTGAGAACAT TGCCTAAGGT TTTGAAGTAT TTACCAAGTG ATAAAGCTCA 960
AGATGCTAGG TTGTACATAC TAAGTTTGCA GTTTTGGCTA GGAGGTTCAC CTGATAATTT 1020
GGTGAATTTC TTGAAAATGA TTTCTGGTTC TTATGTTCCT GCTCTTAAAG GGATGAAAAT 1080
CGACTACTCG GATCCGGTTT TGTACTTGGA TAATGGAATT TGGCACCCTT TGGCTCCTTG 1140
TATGTATGAT GATGTGAAGG AGTATTTGAA TTGGTATGCA ACAAGGAGAG ATACTAATGA 1200
GAAACTCAAG AGTTCAAATG CTCCTGTTGT TGGGCTGGTT TTGCAAAGGA GTCATATTGT 1260
TACTTGTGAT GAGAGTCACT ATGTGGCTGT GATCATGGAA TTGGAGGCAA AGGGGGCTAA 1320
AGTTATCCCA ATTTTTGCCG GTGGGCTAGA CTTTTCGAGG CCAATTGAGA GATATTTCAT 1380
TGATCCTATT ACAAAGAAGC CTTTTGTGAA TTCAGTAATA TCACTTTCTG GTTTTGCACT 1440
TGTTGGAGGG CCAGCAAGAC AAGACCATCC AAGGGCAATA GAGGCTTTGA TGAAACTTGA 1500
TGTGCCTTAT ATTGTGGCAT TGCCTTTGGT TTTCCAAACA ACAGAGGAAT GGTTGAACAG 1560
TACTTTGGGG CTGCACCCTA TTCAGGTGGC TCTACAAGTT GCTCTCCCTG AGCTGGATGG 1620
AGGAATGGAG CCCATCGTAT TCGCCGGTCG CGATCCAAGA ACAGGGAAAT CACATGCTCT 1680
TCACAAAAGA GTGGAGCAGC TTTGCACCAG GGCAATCAAA TGGGGAGAGT TAAAGAGAAA 1740
AACAAAGGCT GAGAAGAGGT TGGCAATCAC TGTCTTCAGC TTTCCTCCAG ACAAAGGCAA 1800
TGTCGGAACT GCTGCATACT TGAATGTCTT TGCCTCCATA TACTCTGTTC TCAAAGATCT 1860
CAAGAAAGAC GGCTACAACG TTGAGGGGCT GCCTGAGACT TCTGCACAAC TTATTGAAGA 1920
AGTAATTCAC GACAAAGAAG CTCAGTTCAG CAGCCCAAAT CTTAACATAG CTTACAAGAT 1980
GAATGTTAGA GAATACCAGA AGCTAACCCC CTATGCTACT GCTCTTGAAG AAAACTGGGG 2040
GAAAGCACCT GGTAATTTGA ACTCTGATGG AGAAAACCTC TTGGTATATG GTAAACAGTA 2100
CGGCAATGTC TTTATCGGTG TTCAGCCCAC GTTTGGATAC GAGGGTGACC CGATGAGACT 2160
TCTGTTCTCC AAATCAGCTA GCCCTCACCA TGGTTTTGCT GCATACTATT CCTTTGTGGA 2220
GAAAATTTTC AAAGCTGATG CAGTTCTCCA CTTTGGTACT CATGGTTCTC TTGAGTTCAT 2280
GCCAGGTAAA CAGGTGGGAA TGAGCGATGC TTCTTTCCCT GATAGTCTCA TTGGAAACAT 2340
TCCCAATGTC TATTACTATG CAGCAAACAA CCCATCTGAA GCAACTATTG CCAAACGAAG 2400
GAGTTATGCG AATACCATTA GCTACTTGAC TCCTCCGGCT GAGAATGCTG GACTCTACAA 2460
GGGACTCAAG CAGCTCAGTG AGCTCATTTC CTCATACCAA TCTCTGAAAG ACTCAGGCCG 2520
TGGCCAACAG ATTGTGAACT CTATCATCAG TACAGCTAGA CAGTGTAATC TTGACAAGGA 2580
TGTTGATCTT CCAGAAGAAG GGGAGGAAAT CTCGGCCAAA GAGCGTGACC TTGTGGTAGG 2640

AAAAGTATAC TCTAAGATTA TGGAGATCGA GTCTCGTCTT CTTCCGTGTG GACTTCACAT 2700
CATTGGTGAA CCTCCAACCG CGATGGAAGC AGTTGCTACT CTTGTCAATA TTGCGACATT 2760
GGACCGTCCT GAAGAGGGTA TTTCTGCCCT TCCATCTATA TTGGCTGCGA CGGTTGGAAG 2820
AAGCATTGAG GAGATTTACA GAGGCAATGA CCAGGGCATC TTACGAGATG TGGAGCTGCT 2880
CCGTCAAATT ACTGAGGCAT CACGTGGAGC AATATCAGCA TTTGTTGAAC GTACGACAAA 2940
CAACAAGGGT CAGGTTGTGA ATGTCAATGA CAAGCTAACC TCAATCCTTG GTTTTGGTAT 3000
AAATGAACCA TGGATCCAGT ATTTGTCAAA CACCCAATTT TACAGAGCTG ATAGGGACAA 3060
GCTCAGAGTT CTATTCCAGT TCTTGGGAGA GTGTCTGAAG CTAATTGTCG CTAACAACGA 3120
GGTGGGAAGC TTGAAACAGG CTCTAGAAGG GAAATATGTT GAACCAGGTC CAGGAGGGGA 3180
TCCGATCAGA AACCCGAAAG TTTTGCCTAC TGGGAAAAAC ATCCATGCTT TGGACCCACA 3240
AGCTATTCCC ACAATAGCAG CAGTGCAGAG TGCCAAAATT GTTGTTGAAA GATTGTTGGA 3300
GAGGCAAAAG GCCGACAACG GGGGCAAGTA CCCGGAGACT GTTGCTCTGG TTCTTTGGGG 3360
AACAGACAAC ATCAAGACCT ATGGAGAGTC ATTGGCACAG GTTATGTGGA TGATTGGTGT 3420
TAGGCCAGTT ACAGACTCGT TAGGACGGGT TAACCGGGTG GAACCTGTTA GCCTTGAAGA 3480
GCTTGGAAGG CCTAGAGTTG ATGTTGTTGT CAACTGCTCT GGGGTGTTCA GAGATCTCTT 3540
CATCAATCAG ATGAATCTCC TTGACCGAGC AGTCAAGATG GTTGCAGAGC TCGACGAGCC 3600
AGAAGACCAA AACTACGTCA GGAAACATGC ACTAGAACAA GCAAAAACAC TCGGAGTTGA 3660
TGTTCGTGAA GCTGCTACAA GGATCTTCTC AAATGCTTCA GGATCTTACT CCTCCAACAT 3720
TAACCTTGCT GTTGAGAATT CAACATGGAA TGATGAGAAG CAACTTCAAG ACATGTACTT 3780
GAGCCGAAAG TCATTTGCAT TTGACTGTGA TGCCCCTGGT GTTGGCATGA CTGAGAAGAG 3840
GAAAGTTTTT GAGATGGCTC TTAGCACGGC TGATGCCACA TTCCAGAACC TTGACTCATC 3900
TGAAATTTCA TTCACAGACG TGAGTCACTA CTTCGATTCA GACCCAACCA ACCTTGTGCA 3960
AAACCTCAGG AAAGACGGGA AGAAGCCTAG TGCATACATT GCTGACACCA CTACTGCTAA 4020
TGCTCAGGTA CGTACGTTGT CTGAGACTGT GAGGCTTGAC GCAAGGACAA AGTTGTTGAA 4080
CCCCAAGTGG TATGAAGGCA TGCTATCCAC TGGCTACGAG GGTGTTCGTG AGATTGAGAA 4140
ACGATTAACT AACACAGTGG GGTGGAGTGC AACTTCAGGC CAAGTTGATA ATTGGGTGGA 4200
TGAAGAAGCC AACACAACCT TCATTCAAGA TCAGGAGATG TTGAACAGGC TCATGAACAC 4260
AAATCCAAAT TCTTTCAGGA AGTTGCTTCA GACATTCTTG GAAGCCAACG GGCGTGGATA 4320
CTGGGAAACT TCTGCAGAGA ACATTGAGAA ACTCAAGCAA TTATACTCAG AAGTTGAAGA 4380
CAAGATTGAG GGAATCGATC GATAAATGTA TAGCAAAAAG AATGATCTCT GATTATTGCC 4440
TGTTTGTTCC TAACTGTTTC TGATGTGAAT TCCTTTGACA GTCCCCAGTG TAATTTTGTT 4500
CATTTTTGGG GATGTCCTAC TTCTATGAGA AAATACTGCT TCCATATATT CAAATTTGAG 4560

CTTGAAAAAA AAAAAAAA 4578

2) INFORMATION ZU SEQ ID NO: 7:

(i) SEQUENZ CHARAKTERISTIKA:

(A) LÄNGE: Aminosäuren
(B) ART: Aminosäure
(C) STRANGFORM: Einzel
(D) TOPOLOGIE: linear

(ii) ART DES MOLEKÜLS: Protein

(ix) MERKMALE:

(A) NAME/SCHLÜSSEL: Protein
(B) LAGE: 1..6

(xi) SEQUENZBESCHREIBUNG: SEQ ID NO: 7:

Ala Lys Gly Ala Val Met